# EUROPÄISCHE PATENTSCHRIFT

(11) **EP 1 635 885 B1**
(45) Veröffentlichungstag und Bekanntmachung des Hinweises auf die Patenterteilung: **26.11.2014**
(21) Anmeldenummer: 04740350.6
(22) Anmeldetag: 25.06.2004
(51) Int. Cl.: A61L 24/00, A61L 27/56

(54) **KNOCHENAUFBAUMITTEL UND HERSTELLUNGSVERFAHREN**
OSTEOGENESIS PREPARATION AND ASSOCIATED PRODUCTION METHOD
AGENT D'OSTEOGENESE ET SON PROCEDE DE PRODUCTION

(30) Priorität: 26.06.2003 DE 10328892
(43) Veröffentlichungstag der Anmeldung: 22.03.2006
(73) Patentinhaber: Curasan AG, 65933 Frankfurt/Main (DE)
(72) Erfinder: REIF, Dieter, 65933 Frankfurt/Main (DE); PETERS, Fabian, 65933 Frankfurt/Main (DE); PALM, Frank, 65933 Frankfurt/Main (DE); WITTNER, Joachim, 65933 Frankfurt/Main (DE)
(74) Vertreter: Boeters, Hans Dietrich
(86) Internationale Anmeldenummer: PCT/EP2004/006947
(87) Internationale Veröffentlichungsnummer: WO 2004/112855

(56) Entgegenhaltungen:
- EP-A- 1 197 233
- WO-A-02/083194
- DE-A- 3 133 016
- DE-A- 10 013 378
- DE-A- 10 160 178
- DE-U- 29 922 585
- US-A- 5 384 290
- US-A- 6 153 547
- US-A1- 2002 165 616
- US-B1- 6 210 715

## Beschreibung

Die Erfindung betrifft ein synthetisches, bioresorbierbares Knochenaufbaumittel für die Behandlung von Knochendefekten am menschlichen oder tierischen Skelett. Es dient zur vorübergehenden Auffüllung eines knöchernen Defektes, bildet eine Leitschiene für die knöcherne Regeneration des Defektes und wird simultan zur Knochenneubildung in einem klinisch überschaubaren Zeitraum vom Organismus resorbiert.

Neben Knochenersatz- und Knochenaufbaumitteln biologischem Ursprungs sind auch synthetische Biomaterialien für die Knochendefektfüllung seit vielen Jahren bekannt. Als eine bedeutende Stoffgruppe für diesen Anwendungsbereich haben sich speziell die Calciumphosphate etabliert. Aufgrund der chemischen Ähnlichkeit mit dem mineralischen Bestandteil des Knochens kommt insbesondere den Materialien mit Hydroxylapatitstruktur in gesinterter, aber auch ungesinterter Form, als Granulat oder in Form massiver Formstücke besondere Bedeutung zu. Vor allem in den zurückliegenden 20 Jahren ergänzten die sogenannten "bioaktiven Glaskeramiken" bei den nicht resorbierbaren Implantaten das Sortiment der Werkstoffe für den Knochenersatz.

Generell kann man feststellen, daß die Entwicklung der Biokeramiken und vergleichbarer Werkstoffe in zwei Richtungen verlief, die hinsichtlich klinisch relevanter Indikationen beide ihre Berechtigung haben: im Körper langzeitstabile Materialien, die sich durch eine gute hydrolytische Beständigkeit gegenüber der Körperflüssigkeit auszeichnen und bioabbaubare Materialien, die teils in der Körperflüssigkeit langsam aufgelöst, teils zellulär abgebaut werden, ohne spürbare Fremdkörperreaktionen auszulösen.

Speziell letztere Materialgruppe hat für ein Konzept der Knochenregeneration zunehmende Bedeutung gewonnen, das davon ausgeht, den ursprünglichen, natürlichen Zustand des Knochens vor einer Defektbildung wieder herzustellen, ein Vorgehen, das als "restitutio ad integrum" bezeichnet wird. Knochenneubildung und Bioresorption/Biodegradation des Knochenaufbaumittels laufen dabei simultan in einer Weise ab, daß das Knochenaufbaumittel den Defekt temporär ausfüllt, osteokonduktiv als Leitschiene das knöcherne Durchbauen des Defektes fördert und gleichzeitig möglichst gut der Geschwindigkeit der Knochenneubildung angepaßt, vom Körper resorbiert wird, so daß das Knochenaufbaumittel einerseits voll seine osteokonduktiven Eigenschaften entfalten kann, andererseits aber auch nicht zu einer Wachstumsbarriere für den neuen Knochen wird. Je besser die beiden Prozesse aufeinander abgestimmt sind, desto qualitativ hochwertiger ist ein Knochenaufbaumittel zu bewerten.

Die Regenerationsfähigkeit eines Knochenaufbaumittels wird nicht allein von seiner Materialzusammensetzung bestimmt. Auch chemisch gleich zusammengesetzte Stoffe können ein deutlich unterschiedliches Regenerationspotenzial aufweisen. Der Grund für dieses unterschiedliche Verhalten liegt dann in der Regel in der Mikrostruktur des Biowerkstoffes. So anerkennt man heute die Bedeutung einer interkonnektierenden Mikroporosität sowie die Rolle von Makroporen in einer Biokeramik für eine erfolgreiche knöcherne Integration des Materials ebenso wie auch für sein Resorptionsverhalten. Unter diesem Aspekt wird der Stand der Technik heute durch Biokeramiken repräsentiert, die mikro- und makroporöse Sinterstrukturen aufweisen und dabei Gesamtporositäten von mindestens 50 Vol.-% erreichen.

Zahlreiche Erfindungsbeschreibungen gehen allerdings bereits zu noch höheren Gesamtporositäten. In EP 0267624 wird ein Knochenersatzmaterial auf Calciumphosphat-Basis beschrieben, das bei einer Gesamtporosität von bis zu 75 % offene und geschlossene Poren aufweist, wobei den offenen Poren eine besondere Bedeutung bezüglich der Fremdkörperreaktion des Implantates zukommt. Besonders Poren im Durchmesserbereich von 0,01 bis 50 µm sollen nach einer Beobachtung dazu führen, daß die körpereigenen Abwehrzellen das Material nicht mehr als Fremdkörper identifizieren. Die offenen Poren können einen breiten Bereich der mittleren Größe von 0,01 bis 2000 µm überstreichen.

Mit DE 3717818 wird ein mikroporöses Knochenprothesenmaterial geschützt, das aus porösem Calciumphosphat hergestellt wird. Die Körnchen aus porösem Calciumphosphat weisen offene Zellen gleich oder größer als 0,01 µm und kleiner als 10 µm auf. Die Gesamtporosität kann bis zu 90 % betragen. Auch diesem Material liegt die Beobachtung zugrunde, daß anhaftende Makrophagen das Material nicht als Fremdkörper identifizieren, wenn es von Körperflüssigkeit hinreichend durchspült wird.

Nach DE 29922585 wird ein temporärer Knochendefektfüller beansprucht, der durch interkonnektierend verbundene Mikroporen einer mittleren Größe im Bereich von 0,5 bis 10 µm mit einem Anteil an der Gesamtporosität von 20 bis 50 % und wenigstens teilweise interkonnektierend verbundene Makroporen einer mittleren Größe im Bereich von 50 bis 1000 µm bei einem Anteil an der Gesamtporosität von 50 bis 80 % gekennzeichnet ist, wobei die nicht interkonnektierend verbundenen Makroporen über Mikroporen mit ihren Nachbarn verbunden sind, die Makroporen eine typisch polyedrische Gestalt aufweisen und die Gesamtporosität > 50 Vol.-% beträgt.

Kugelförmige Poren weist ein in DE 3425182 geschütztes Knochenersatzmaterial auf Calciumphosphatbasis mit einer Porosität von 40 bis 90 % auf, wobei die weitgehend kugelförmigen Poren im Größenbereich von 3 bis 600 µm liegen und durch kapillare Porenkanäle mit einem Durchmesser von 1 bis 30 µm untereinander und mit der Oberfläche des Formkörpers verbunden sind. Die Porenkanäle werden durch einen Zusatz von organischen Fasern zur Ausgangsmischung erzielt.

Ebenfalls kugelförmige Poren weist ein Knochenersatzmaterial nach DE 19581649 T1 auf, wobei gleichzeitig auf der Oberfläche des Implantates konkave Vertiefungen zur Anregung des Knochenwachstums vorhanden sind. Die mittleren Porendurchmesser der kugelförmigen Poren liegen im Bereich von 300 bis 2000 µm. Wenigstens ein Teil der Makroporen ist interkonnektierend verbunden. Zusätzliche Mikroporen sind nicht beschrieben.

Nach WO 01/13970 A1 und DE 19940717 A1 werden Formteile aus einem resorbierbaren Knochenersatz- und Knochenaufbaumaterial aus porösem Beta-TCP zum Schutz beansprucht, die eine interkonnektierende Mikroporosität und eine gerichtete Makroporosität in Form von maschinell eingebrachten Röhrenporen aufweisen. Die Röhrenporen sind vorzugsweise in der Knochenwachstumsrichtung orientiert.

US 6521246 schützt anorganische Formkörper aus Calciumphosphat für den Einsatz zur Knochenheilung in Lebewesen mit einer im wesentlichen gleichförmigen Makro-, Meso- und Mikroporosität bei einer Gesamtporosität von mindestens 30 % sowie Verfahren zur Herstellung. Unter Makroporsität werden dabei Poren gleich oder größer 100 µm, unter Mesoporosität Poren mit Durchmessern zwischen 10 und 100 µm und unter Mikroporosität Poren kleiner 10 µm verstanden. Die Gesamtporosität aller Poren kann bis zu 95 % betragen.

Nach WO 02/083194 wird eine osteokonduktive oder osteoinduktive Biostruktur aus miteinander verbundenen Partikeln zum Schutz beansprucht. Die Partikel bilden eine Matrix, die wenigsten einen porösen Teil aufweist und aus bis zu drei Strukturtypen bestehen kann. Die Basisstruktur ist eine Mikrostruktur mit einer unimodalen Porengrößenverteilung bei einer mittleren Porengröße zwischen 10 und 50 µm. Dieser können mittels 3D-Printtechnik die weiteren Strukturtypen Mesostruktur und Makrostruktur zugefügt werden. Eine Biostruktur mit mehreren Strukturtypen weist dann eine bimodale Poregrößenverteilung auf. Als Biostruktur wird nach WO 02/083194 ein über die 3D-Printtechnik erzeugter Formkörper mit genau definierten konstruktiven Merkmalen verstanden.

Ein Verfahren zur Herstellung eines ähnlichen Formkörpers stellt WO 00/42991 unter Schutz. Der nach diesem Verfahren hergestellte Formkörper weist eine im wesentlichen gleichförmige Makro-, Meso- und Mikroporosität bei einer Gesamtporosität von wenigstens 30% auf. Weiterer Schutz wird auf ein Verfahren zur Knochenregeneration unter Verwendung eines Formkörpers mit einer Gesamtporosität von wenigstens 50% erhoben.

Ein weiterer poröser, biokeramischer Formkörper wird nach EP 1197233 geschützt. Es handelt sich um einen Formkörper aus geschäumten Calciumphosphat. In der keramischen Mikrostruktur werden sphärische Poren erzeugt, so dass mittels Quecksilberporosimetrie bei einigen Ausführungsbeispielen eine bimodale Porengrößenverteilung gemessen wurde.

Nach WO 98/15505 wird ein Herstellungsverfahren für poröse, biokeramische Artikel zum Schutz beansprucht, dass ebenfalls ein Schäumverfahren nutzt, den gebildeten keramischen Schaum mittels Polymerisation eines Monomers stabilisiert und die Artikel zur Entfernung der organischen Bestandteile und zur Versinterung der keramischen Partikel brennt.

WO 92/21302 beansprucht ein poröses Implantat zum Schutz, dass aus unterschiedlich porösen Zonen besteht, wobei das Implantat zur Unterstützung einer knöchernen Verwachsung mit dem Knochen an der Oberfläche Makroporen im Bereich von 50 bis 500 µm aufweist.

Ein poröses Hydroxylapatitmaterial ist nach DE 3531144 bekannt, das als Granulat für die Knochendefektfüllung mit einer offenen Mikroporosität mit einer Porengrößenverteilung im Bereich von 10 bis 100 µm mit einer Gesamtporosität im Bereich von 20 bis 50% verwendet wird und als Implantat eine Porengrößenverteilung im Bereich von 200 bis 2000 µm aufweist. Bei mechanisch hohen Ansprüchen kann das Implantat zusätzlich eine Oberflächenschicht aus mikroporösem Material aufweisen.

Nach dem Stand der Technik ist davon auszugehen, daß das Regenerationspotenzial eines Knochenaufbaumittels folglich zu wesentlichen Anteilen durch seine spezielle Morphologie der Porosität bestimmt wird. Während ein interkonnektierendes Mikroporennetzwerk vor allem die Biokompatibilität des Materials sichert, begünstigen interkonnektierende Makroporen in einem Größenbereich von 100 bis 500 µm vor allem das knöcherne Durchbauen des Materials. Dies scheint unabhängig davon zu sein, ob es sich um ein Material synthetischen oder bovinen Ursprungs bzw. ein bioresorbierbares oder nicht bioresorbierbares Knochenaufbaumittel chandelt.

Bei resorbierbaren Knochenaufbaumitteln entsteht durch die Makroporen ein weiterer Vorteil dadurch, daß durch die Reduzierung der Materialdichte pro Defektvolumen eine geringere Materialmenge resorbiert werden muß, was einerseits den Stoffwechsel des Patienten weniger belastet, andererseits sogar eine zeitliche Verkürzung des Resorptionsprozesses bewirkt. Folgebehandlungen, wie das Setzen von Zahnimplantaten nach einer Auffüllung der Alveolen mit einem bioresorbierbaren Knochenaufbaumittel sind somit ganz im Sinne des Patienten zu einem früheren Zeitpunkt möglich.

Die im Stand der Technik genannten strukturellen Merkmale der Porosität allein sind für die Spezifizierung eines bioresorbierbaren Knochenaufbaumittelsjedoch noch nicht ausreichend. Die Festigkeit seiner Sinterstruktur, d.h. die Festigkeit der versinterten Kontaktstellen zwischen den der Sinterung zugeführten Pulverpartikeln des Knochenaufbaumittels und deren Größe sind weitere wichtige Merkmale, die seine Biokompatibilität bestimmen. Die sogenannten Sinterhälse oder Sinternecks zwischen den keramischen Partikeln müssen eine solche mechanische Stabilität aufweisen, daß die Sinterstruktur wenigstens über die Phase der Wundheilung in den ersten Wochen postoperativ erhalten bleibt und die Struktur des Knochenaufbaumittels nicht beim Kontakt mit Körperflüssigkeit partikulär zerfällt. Tritt ein solcher Fall ein und weisen die versinterten Partikel des Calciumphosphates eine Partikelgröße < 10 µm auf, lösen sie zusätzlich zu den mit der Wundheilung verbundenen Entzündungserscheinungen Fremdkörperreaktionen aus, die den Knochenheilungsprozess verzögern oder verhindern können.

Dies gilt im übertragenen Sinne auch für ungesinterte Materialien, nur dass hier die Fixierung der Partikel über andere Mechanismen, wie Polymerbindung (DE 19614421 A1) oder Maskierung durch ein Xerogel (z.B. WO 01/54747 A1) erfolgt. Auch in diesen Fällen muss eine spontane Freisetzung von Partikeln aus den genannten Gründen vermieden werden bzw. diese Freisetzung auf eine klinisch tolerable Menge von Partikeln, die noch keine akuten Entzündungsreaktionen hervorrufen, begrenzt werden.

Ein weiteres wichtiges Qualitätskriterium für ein gesintertes Knochenaufbaumittel ist folglich eine stabile Sinterstruktur mit festen Sinterhälsen von Calciumphosphatpartikel zu Calciumphosphatpartikel, die Partikelfreisetzungen erst im Zusammenhang mit dem Resorptionsprozeß zulassen. Nicht in jedem Falle sind allerdings während der Resorption freigesetzte Partikel unkritisch. Wie von Klein et al. gefunden wurde, können schwer lösliche Partikel phagozytär abtransportiert werden und lagern sich im Lymphsystem des Körpers ab (Biomaterials, 6 (1985) 189-192). Über die Langzeitwirkung solcher kristalliner Partikel in den Lymphknoten gibt es derzeit noch keine gesicherten Erkenntnisse. Solche Fälle treten bevorzugt dann auf, wenn bioresorbierbare Knochenaufbaumittel aufgrund von Unzulänglichkeiten des Herstellungsverfahrens nicht phasenrein hergestellt werden und schwer oder nicht bioresorbierbare Phasenbestandteile beinhalten. Dies war speziell in der Anfangszeit der Anwendung von Beta-Tricalciumphosphat zur Knochendefektfüllung zu verzeichnen. Abweichungen von der Stöchiometrie oder ungeeignete Prozeßführung führten zu erheblichen Anteilen von Hydroxylapatit als Fremdphase im Tricalciumphosphat. Wegen seiner Schwerlöslichkeit im gesinterten Zustand bleibt Hydroxylapatit im Verlaufe der Resorption des Tricalciumphosphates in partikulärer Form zurück, wird phagozytär aus dem Defekt entfernt und findet sich, wie die Untersuchungen von Klein et al. zeigen, in den umliegenden Lymphknoten wieder.

Somit ergibt sich für die Qualitätsbeurteilung speziell des Knochenaufbaumittels Beta-Tricalciumphosphat der Grad der Phasenreinheit als ein weiteres, bedeutendes Kriterium. Selbst wenn die ASTM F 1088 - 87 (Reapproved 1992) "Standard Specification for Beta-Tricalcium Phosphate for Surgical Implantation" einen Hydroxylapatitgehalt von kleiner/gleich 5 Masse-% im Beta-Tricalciumphosphat zuläßt, muss unter dem Aspekt der oben beschriebenen Risiken ein Beta-Tricalciumphosphat als um so hochwertiger eingestuft werden, je geringer der Anteil an dieser Fremdphase ist.

Moderne Fertigungsverfahren gestatten heute die Herstellung von Beta-Tricalciumphosphat mit einer Phasenreinheit bezüglich Hydroxylapatit von besser als 99 Masse-%, also mit Gehalten an dieser Phase deutlich unter 1 Masse-%. Solche weitgehend phasenreinen Produkte sind für Anwendungen zur Implantation im menschlichen Körper in jedem Falle solchen mit deutlich nachweisbaren Anteilen an Hydroxylapatit vorzuziehen, um die genannten Risiken auszuschließen.

Selbstverständlich spielt die chemische Zusammensetzung eines bioresorbierbaren Knochenaufbaumittels hinsichtlich seiner Resorptionsgeschwindigkeit eine entscheidende Rolle. Unter den synthetischen, bioresorbierbaren Knochenaufbaumitteln hat sich vor allem in den zurückliegenden 10 bis 15 Jahren das Tricalciumphosphat, insbesondere das Beta-Tricalciumphosphat durchgesetzt. WO 91/07357 beschreibt u.a. Knochenaufbaumittel mit verbesserter Resorbierbarkeit. Das Ziel besteht in einer Verkürzung der Resorptionszeit bei einer gleichzeitigen Beschleunigung der Knochenregeneration. Bei den zum Schutz beanspruchten Materialien handelt es sich um chemisch modifiziertes Tricalciumphosphat, bei dem ein Teil der Calciumionen zur Verbesserung der Löslichkeit durch andere Kationen ersetzt wird.

Dass nicht allein die chemische Komposition des Knochenaufbaumittels für seine Regenerationseigenschaften verantwortlich ist, erkennt man speziell beim Tricalciumphosphat sehr gut. Tricalciumphosphat der Summenformel Ca₃(PO₄)₂ kann je nach Herstellungsbedingungen in zwei unterschiedlichen Kristallmodifikationen hergestellt werden, einer Hochtemperatur- oder Alpha-Form und einer Tieftemperatur- oder Beta-Form. Chemisch besteht zwischen diesen beiden Modifikationen keinerlei Unterschied. Dennoch verhalten sich die beiden Modifikationen bei sonst gleichen Merkmalen, wie Sinterstruktur, Porosität, Größe der Sinterpartikel und Festigkeit der Sinterhälse im Kontakt mit der Körperflüssigkeit völlig unterschiedlich.

Die bei Raumtemperatur metastabile Hochtemperaturmodifikation Alpha-TCP stellt eine energiereichere Zustandsform dar, die sich aus energetischen Gründen und wegen ihrer kristallographischen Analogie im Kontakt mit Körperflüssigkeit in Hydroxylapatit umlagert (Lin et al., Biomaterials, 22 (2001) 2990). Wegen der schlechteren Löslichkeit des Hydroxylapatits verlängert diese Phasenumwandlung die Resorptionszeit des Alpha-TCP deutlich, obwohl diesem eine höhere Löslichkeit als dem Beta-TCP zugeschrieben wird (Lin et al., Biomaterials, 22 (2001) 2981). Die energetisch stabilere Tieftemperaturmodifikation Beta-TCP zeigt diese Phasenumwandlung nicht und wird deshalb, vergleichbare Sinter-, Porenstruktur und Implantatlageraktivität vorausgesetzt, schneller als die Alpha-Modifikation resorbiert.

Die bekannten Mittel zur Regeneration von Knochendefekten sind vor allem hinsichtlich der Verbesserung ihrer Sinter- und Porenstruktur im Granulatkorn beschrieben. Man beobachtet hier einen Trend zu zunehmend höheren Porositäten, wobei sich die Gesamtporosität von Granulaten aus Anteilen von Mikro- und Makroporosität zusammensetzt. Unter Mikroporosität werden dabei Porengrößenverteilungen < 10 µm verstanden, Makroporosität beginnt dagegen bei Porengrößen über 100 µm. Der Stand und die Entwicklung mikro- und makroporöser Knochenregenerationsmittel ist heute durch einen zunehmenden Anteil an Makroporen charakterisiert, was die mechanische Stabilität der Sinterstrukturen immer mehr schwächt. Dadurch entsteht für derartige Knochenregenerationsmittel ein immer größeres Risiko, dass sie den mechanischen Beanspruchungen des Transportes und der Applikation in den Defekt nicht mehr standhalten, partikulär zerfallen und ggf. dadurch sogar Fremdkörperreaktionen auslösen.

Die Kornform des Granulates kann verschiedene Geometrien aufweisen. Bekannt sind vor allem eine kugelige, eiförmige und polygone Gestalt. Für die praktische klinische Anwendung werden die Granulate üblicherweise in Kornbändern von 50 bis 2000 µm bereitgestellt, in einzelnen Fällen auch darüber. Für spezielle Indikationen wird das Kornband weiter unterteilt, z.B. 50 bis 150 µm, 150 bis 500 µm, 500 bis 1000 µm und 1000 bis 2000 µm.

Beliebig zunehmenden Materialporositäten sind bezüglich der Festigkeit Grenzen gesetzt, die eine weitere Reduzierung der Menge des Knochenaufbaumittels pro Defektvolumen beschränkt. Je höher die Gesamtporosität in Form von Mikro- und Makroporen gezüchtet wird, umso geringer ist die mechanische Festigkeit, die die Granulatkörner aufweisen. Auf keinen Fall darf das Einbringen des Granulates in den Defekt oder das Anmischen des Granulates z.B. mit Patienteneigenblut oder PRP (Platelet Rich Plasma) die Granulatstruktur zerstören. Dies setzt der Gesamtporosität eines Knochenaufbaumittels Grenzen, da eine solche Zerstörung der Struktur aufgrund von Feinpartikelbildung zu Knochenheilungsproblemen durch Fremdkörperreaktion führt.

Zusätzlich deuten Hinweise aus der klinischen Praxis auf einen bisher wenig beachteten Sachverhalt bei Calciumphosphatkeramiken mit interkonnektierenden Makroporensystemen hin. Ein oberflächlich zugängliches und wenigstens teilweise interkonnektierendes Makroporensystem in den Granulatkörnern kann nach dieser Beobachtung einen Unterschlupf für Keime bilden und das Risiko für eine erfolgreiche Knochenregeneration im Defekt erhöhen. Dringen Keime in derartige Positionen ein, sind sie einer systemischen Behandlung mit Antibiotika schwer oder nicht zugänglich. (Palm, F.: Calcium phosphate ceramics as a bone substitute material - A prospective clinical trail. IMOI, submitted)

Das Ziel der Erfindung besteht darin, den Stand der Technik zu verbessern, insbesondere eine hinreichende mechanische Festigkeit des Knochenaufbaumittels bei hoher Gesamtporosität zu sichern und Risiken in Problemdefekten zu verringern.

Der Erfindung liegt die Aufgabe zugrunde, bei vorgegebener, für eine Knochendefektfüllung hinreichender mechanischer Festigkeit des Knochenaufbaumittels durch ein neues Porositäts- und Sinter-Design ohne interkonnektierende Makroporosität die knöcherne Regeneration des Defektes zu verbessern.

Die erfindungsgemäße Aufgabe wird durch die Schaffung eines neuen Knochenaufbaumittels aus porösem Calciumphosphat mit isotroper Sinterstruktur und statistisch zwischen den versinterten Partikeln des Calciumphosphates verteilten Poren in mehreren diskreten Größenbereichen gemäß Anspruch 1 gelöst. Das Knochenaufbaumittel weist eine Porosität mit einer irregulären polygonen geometrischen Gestalt auf und besitzt wenigstens drei diskrete, Porengrößenverteilungen (I), (II) und (III) mit ausgeprägten Maxima. Die Größe der miteinander versinterten Calciumphosphatpartikel liegt bei kleiner 63 µm mit einem d₅₀-Wert im Bereich von 5 bis 20 µm. Die Poren bilden den leeren Raum zwischen den Partikeln des Calciumphosphates, wobei der interkonnektierende Porenanteil auf Porengrößen kleiner 10 µm begrenzt ist.

Die Ausführungsform der Erfindung weist drei Maxima der Porengrößenverteilung (I), (II) und (III) auf. In diesem Falle liegen die Maxima der Porengrößenverteilungen bei Porendurchmessern in den Bereichen 0,5 bis 10 µm für die Porengrößenverteilung (I), 10 bis 100 µm für die Porengrößenverteilung (II) und 100 bis 5000 µm für die Porengrößenverteilung (III).

Die gewählte Größe der versinterten Partikel des Calciumphosphates ist, verglichen mit dem Stand der Technik mit < 63 µm und einem d₅₀-Wert von 5 bis 20 µm relativ groß gewählt, so dass mehr als 50 % der Partikel über der durch Makrophagen zugänglichen Größe (< 5 µm) liegen. Dies ist neben einem festen keramischen Verbund durch stabile Sinterhälse eine zusätzliche Sicherheit, um Fremdkörperreaktionen zu vermeiden.

Zur Beschleunigung des Resorptionsprozesses wird nach dem Stand der Technik versucht, das Materialangebot pro Defektvolumen durch die Erzeugung einer großen Zahl von möglichst interkonnektierenden Makroporen in einem für das Knocheneinwachsen relevanten Größenbereich von 100 bis 2000 µm zu reduzieren. Eine zunehmende Zahl an Makroporen wirkt sich jedoch einerseits negativ auf die Materialfestigkeit aus und erhöht andererseits das Risiko für die Anwendung des Knochenaufbaumittels in sogenannten Problemdefekten. Demgegenüber wird erfindungsgemäß ein interkonnektierendes Makroporennetzwerk ausgeschlossen und werden die Größen/Kanalquerschnitte eines interkonnektierenden Porensystems nach oben hin auf 10 µm begrenzt. Gleichzeitig wird durch Modifizierung des Herstellungsverfahrens eine verbesserte Festigkeit der Sinterhälse erzielt.

Charakteristisch für das erfindungsgemäße Knochenaufbaumittel ist, dass das Mengenverhältnis der Poren in den drei Porengrößenverteilungen gezielt eingestellt werden und an den Anwendungszweck angepasst werden kann. Mit der Einstellung dieses Mengenverhältnisses der unterschiedlichen Porengrößen wird auch vermieden, dass der Anteil an statistischen Poren der Porengrößenverteilung (III) über ein bestimmtes Maß ansteigt und diese unter Umständen interkonnektierend werden. Neben oder an Stelle statistischer Poren der Porengrößenverteilung (III) kann in Formkörpern definierter geometrischer Abmessung zusätzlich eine gerichtete Porosität in Form von Röhrenporen vorliegen. Diese Röhrenporen werden durch eine maschinelle Bearbeitung eingebracht. Sie können eine Orientierung in einer, zwei oder drei Raumrichtungen vorliegen und reichen üblicherweise von einer Oberfläche des Formkörpers bis zur gegenüberliegenden. Röhrenporen in einer Raumrichtung sind vorzugsweise parallel angeordnet. Sind sie in mehrere Raumrichtungen orientiert, bilden sie miteinander rechte Winkel und können sich kreuzen. Bevorzugt ist eine Anordnung der Röhrenporen in Knochenwachstumsrichtung.

Zur Erzielung optimaler Eigenschaften des Knochenaufbaumittels liegen die Volumenanteile der Porengrößenverteilungen (I) bis (III) in bestimmten prozentualen Anteilen an der Gesamtporosität vor. Bewährt haben sich für gute Materialfestigkeiten bei Ausschluss interkonnektierender Makroporosität für die Porengrößenverteilung (I) ein Anteil im Bereich von 20 bis 40 Vol.-%, für die Porengrößenverteilung (II) ein Anteil von 5 bis 40 Vol.-% und für die Porengrößenverteilung (III) ein Anteil im Bereich von 1 bis 40 Vol.-%, wobei die Gesamtporosität mit 85 Vol.-% begrenzt ist, um hinreichende Anwendungsfestigkeiten zu gewährleisten.

Nach diesem Konzept sind Poren der Porengrößenverteilungen (II) und (III) ausschließlich über das interkonnektierende Porensystem (I) miteinander und mit der Oberfläche verbunden, so dass es unmöglich ist, dass das Innere des Knochenaufbaumittels durch Keime besiedelt wird und diese sich dadurch einer systemischen Behandlung mit Antibiotika entziehen können.

Das erfindungsgemäße Knochenaufbaumittel kann aus beliebigen, zur Knochenregeneration geeigneten Materialien aufgebaut sein, besteht aber bevorzugt im wesentlichen und insbesondere zu wenigstens 95 % aus einem Calciumphosphat der Gruppe Alpha-Tricalciumphosphat, Beta-Tricalciumphosphat, Octacalciumphosphat, alkali- und/oder erdalkalimodifiziertem Tricalciumphosphat, Calciumdiphosphat, Carbonatapatit vom B-Typ, Calcium-defizienten Hydroxylapatit oder deren Gemischen. Eine besondere Ausführungsform der Erfindung bezieht sich auf ein Knochenaufbaumittel aus Calciumphosphat, vorzugsweise auf ein zu 99 oder mehr als 99 Masse-% phasenreines Beta-Tricalciumphosphat, bezogen auf die Fremdphase Hydroxylapatit.

Das erfindungsgemäße Knochenaufbaumittel kann in Form geeigneter Granulate zur Knochendefektfüllung eingesetzt werden. Gängige Korngrößenverteilungen für solche Granulate liegen im Bereich von 50 bis 10000 µm, bevorzugt im Bereich von 50 bis 8000 µm. In der Regel werden indikationsbezogen engere Korngrößenbereiche zum Einsatz gebracht, z.B. 50 bis 150 µm, 150 bis 500 µm, 500 bis 1000 µm, 1000 bis 2000 µm usw.

Die Granulate des erfindungsgemäßen Knochenaufbaumittels können je nach Anwendung eine im wesentlichen uneinheitliche, irreguläre, polygone geometrische Gestalt aufweisen, sie können aber auch eine im wesentlichen einheitliche geometrische Gestalt, zum Beispiel Kugelgestalt besitzen.

Ein weiteres erfindungsgemäßes Merkmal des Knochenaufbaumittels in Form von Granulat ist eine Abstimmung des Porendurchmessers auf den Granulatdurchmesser. So erreicht man günstige mechanische Eigenschaften für Granulate, wenn die mittleren Porendurchmesser im oberen Größenbereich bzw. bei der Porengrößenverteilung (III) 50 % der mittleren Granulatgröße der jeweiligen Granulatfraktion nicht überschreiten. So liegt das Maximum der Porengröße der Porengrößenverteilungen (III) bevorzugt in einem Größenbereich von 10 bis 50 % der mittleren Granulatgröße einer Granulatfraktion.

Die Anforderungen an das Knochenaufbaumittel differieren je nach Verwendung als Granulat oder als Formteil. Da an Formteile in der Regel höhere mechanische Ansprüche als an Granulate gestellt werden, werden hier erfindungsgemäß die Anteile der Porengrößenverteilung (III) zugunsten der Porengrößenverteilung (II) verringert. Bei speziellen Ausführungsformen des Knochenaufbaumittels als Formteil wird sogar auf gerichtete Poren in Form von Röhrenporen völlig verzichtet.

Neben einer granulären geometrischen Gestalt kann das Knochenaufbaumittel auch als Formteil mit einer genau definierten geometrischen Gestalt vorliegen. Das Knochenaufbaumittel als Formteil mit spezieller Geometrie wird durch spanende Bearbeitung auf computergesteuerten Maschinen gefertigt. Auf diese Weise sind beliebige geometrische Formen herstellbar, vorzugsweise Würfel, Quader, Zylinder, Keile und ähnliche Teile. Die gesinterten Rohteile können allerdings auch zur Fertigung individueller Implantate für einen ganz bestimmten Patienten oder für indikationsbezogene Implantate, z.B. Trepanationsverschlüsse, Füllkörper für Cages in der Wirbelsäulenchirurgie, Alveolar-Augmentate für den Dentalbereich und anderes mehr herangezogen werden. Hier sind der Formenvielfalt praktisch keine Grenzen gesetzt.

Zusätzlich zur statistischen Porosität mit diskreten Porengrößenverteilungen ist es auch möglich, durch spanende Bearbeitung eine gerichtete Porosität in Form von Röhrenporen in Formkörper einzubringen. Die Orientierung dieser Röhrenporen wird vorzugsweise der Knochenwachstumsrichtung angepasst und fördert so das knöcherne Durchbauen des Formkörpers. Je nach mechanischer Anforderung an den Formkörper können ein-, zwei- oder auch dreidimensionale Bohrmuster in den Formkörper eingebracht werden. Vorzugsweise liegt diese gerichtete Porosität in einem Durchmesserbereich von 0,5 bis 2 mm. Die Gesamtporosität an statistischer und gerichteter Porosität sollte aus Festigkeitsgründen nicht über 85 Vol.-% liegen.

Erfindungsgemäß ist die Porengrößenverteilung über den Querschnitt eines Formkörpers konstant und/oder variabel. Zur Steigerung der mechanischen Festigkeit eines Formkörper bei angemessener Gesamtporosität ist dieser in einem Randbereich von dichter Struktur aufgebaut und enthält nur Poren der Porengrößenverteilung (I) und/oder (II), während sein Inneres erfindungsgemäß eine Kombination aller Formen der statistischen Porosität aufweist. Die Struktur eines solchen Formkörpers ist dann der Natur des natürlichen Knochens angenähert. Je nach mechanischer Beanspruchung kann einem solchen Formkörper zusätzlich gerichtete Röhrenporosität in ein-, zwei- oder dreidimensionaler Form überlagert werden. Die Gesamtporosität in der Randzone soll dabei einen Wert von 35 Vol.-% nicht übersteigen, während im Innern des Formkörpers die Gesamtporosität mit 85 Vol.-% begrenzt wird.

Die Dicke der Zonen ist variabel und umfasst einen Bereich von 10 bis 40 % des Größtmaßes senkrecht zur Zug- oder parallel zu Biegebeanspruchung des Formteiles für die Randzone und 60 bis 90 % für die Kernzone.

In einer speziellen Ausführungsform der Erfindung ist es auch möglich, das Knochenaufbaumittel in Kombination mit verschiedenen Wirkstoffen, z.B. antibakteriellen Substanzen, Stoffen, die die Wundheilung fördern, wie PRP, Hyaluronsäure u.a., knochenwachstumsfördernden Wirkstoffen und/oder gerinnungshemmenden Wirkstoffen, wie Heparin zu nutzen. Kombinationen verschiedenster Art sind hier vorstellbar. Die Wirkstoffe können auf der Oberfläche aufgetragen werden und entfalten dann eine kurze Wirkungsphase, sie können aber auch das gesamte Porengerüst ausfüllen und dann aufgrund der hohen Kapillarkräfte über eine längere Zeit wirken. Die Art der Anwendung und die Auswahl des Wirkstoffes oder der Wirkstoffkombination erfolgt hier vorzugsweise indikationsbezogen.

Gegenstand der Erfindung ist weiterhin ein Verfahren zur Herstellung eines Knochenaufbaumittels mit isotroper Sinterstruktur und statistisch zwischen den versinterten Partikeln verteilten Poren in mehreren diskreten Größenverteilungen auf Basis von Calciumphosphat gemäß Anspruch 17. Das Verfahren beruht auf einem Syntheseweg über eine thermisch induzierte Festkörperreaktion aus an sich bekannten Ausgangsstoffen. Zur Herstellung werden einer Mischung eines vorab synthetisierten Calciumphosphates mit einem Anteil eines Gemisches seiner unreagierten Ausgangsstoffe nach intensiver Homogenisierung wenigstens zwei ausbrennbare Porosierungsmittel in einer Menge und Kornverteilung so zugesetzt, dass sie jeweils den Anteil einer der zwei gewünschten, diskreten Porengrößenverteilungen erhöhen oder erzeugen. Die Calciumphosphatbestandteile und Porosierungsmittel werden ohne weitere Kornzerkleinerung homogen vermischt, kompaktiert, die Porosierungsmittel werden durch Erhitzen entfernt und die porösen ungebrannten Körper auf Reaktions-/Sintertemperatur über die erforderliche Zeit erhitzt. Die gebrannten Körper werden nachfolgend auf Raumtemperatur abgekühlt und das erhaltene poröse Calciumphosphat entsprechend der gewünschten Granulatgröße zerkleinert oder zu Formkörpern verarbeitet.

Das Verfahren nutzt drei, in ihrem Mengenanteil und ihrer Korngrößenverteilung abgestufte Kornfraktionen an ausbrennbaren Porosierungsmitteln, um im porösen Calciumphosphat Poren in drei diskreten Porengrößenverteilungen zu erzeugen.

Vorzugsweise handelt es sich bei dem Calciumphosphat um Beta-Tricalciumphosphat mit einer Phasenreinheit bezüglich Hydroxylapatit größer gleich 99 Masse-%. Bei den unumgesetzten Ausgangsstoffen (A) und (B) handelt es sich in diesem Falle um Calciumcarbonat CaCO₃ und Calciumhydrogenphosphat CaHPO₄ bei Ausgangsstoff (C) um bereits synthetisiertes Beta-Tricalciumphosphat. Das synthetisierte Beta-Tricalciumphosphat wird vor dem Mischen mit den unumgesetzten Ausgangsstoffen (A) und (B) im Molverhältnis 1:2 auf eine Partikelgröße < 63 µm mit einen d₅₀-Wert im Bereich von 5 bis 20 µm zerkleinert. Mit einem solchen Kornband wird gesichert, dass der Hauptteil der der Sinterung zugeführten Pulverpartikel oberhalb des phagozytierbaren Größenbereiches liegt. Der verbleibende Feinanteil führt im Zusammenspiel mit dem Anteil an unreagierten Ausgangskomponenten zu einem festen Sinterverbund der Partikel des Beta-Tricalciumphosphates.

Um zum Beispiel Tricalciumphosphat mit isotroper Sinterstruktur und statistisch verteilten Poren in mehreren diskreten Größenverteilungen über den Syntheseweg einer thermisch induzierten Festkörperreaktion aus an sich bekannten Ausgangsstoffen herzustellen, geht man vom vorab synthetisierten Ausgangsstoff (C) aus, setzt diesem einen entsprechenden Anteil des Gemisches seiner unumgesetzten Ausgangsstoffe (A) und (B) im Molverhältnis 1:2 zu, homogenisiert die Mischung intensiv und setzt nachfolgend wenigstens zwei (Ausgangsstoffe (D) und (E)), vorzugsweise drei ausbrennbare Porosierungsmittel (Ausgangsstoffe (D) bis (F)) in einer Menge und Kornverteilung so zu, dass sie jeweils den Anteil der gewünschten Anzahl an diskreten Porengrößenverteilungen erhöhen oder erzeugen. Die Mischung der Calciumphosphatbestandteile und Porosierungsmittel wird ohne weitere Kornzerkleinerung homogenisiert, kompaktiert, die Porosierungsmittel durch Erhitzen entfernt und die porösen Körper einem Reaktions-/Sinterbrand zugeführt.

Die Zugabe eines Anteils der Mischung derunumgesetzten Ausgangsstoffe (A) und (B) im Molverhältnis 1:2 zum vorab synthetisierten Ausgangsstoff (C) dient einerseits der Verstärkung der interkonnektierenden Porosität mit der Porengrößenverteilung (I), andererseits aber auch zur Verbesserung des Sinterverhaltens des vorab synthetisierten Ausgangsstoffes (C) und damit zu einer verbesserten mechanischen Festigkeit des porösem Beta-Tricalciumphosphates und wird erfindungsgemäß in einem Mengenanteil im Bereich von 1 bis 50 Masse-%, bezogen auf die Menge an eingesetztem Ausgangsstoff (C) der Mischung zugegeben Je nach Anwendungszweck des Knochenaufbaumittels werden der Mischung des vorab synthetisierten Ausgangsstoffes (C) mit dem Anteil des Gemisches seiner unumgesetzten Ausgangsstoffe (A) und (B) im Molverhältnis 1:2 zusätzlich ausbrennbare Porosierungsmittel in definierter Korngrößenverteilung und Mengenanteil zur Erzielung der gewünschten Anteile an Poren in den Größenverteilungen (I), (II) und (III) zugesetzt.

Als Porosierungsmittel kommt Ammoniumhydrogencarbonat in Betracht, daß sich gut zerkleinern und in diskrete Kornverteilungen aufarbeiten lassen. Aufgrund ihrer Vorgeschichte haben die Porosierungsmittel eine im wesentlichen uneinheitliche geometrische Gestalt, die als irregulär oder polygon zu bezeichnen ist und ihre Größenverteilung liegt in dem gewünschten Porengrößenbereich, da sie beim Ausbrennen einen Hohlraum hinterlassen, der im wesentlichen ihrer ursprünglichen Gestalt und Größe entspricht. Für die Porengrößenverteilung (I) kommt neben dem unreagierten, stöchiometrischen Gemisch der Ausgangsstoffe ein Porosierungsmittel mit einem d₅₀-Wert im Bereich von 0,5 bis 10 µm, für die Porengrößenverteilung (II) ein Porosierungsmittel mit einem d₅₀-Wert im Bereich von 10 bis 100 µm und für die Porengrößenverteilung (III) ein Porosierungsmittel mit einem d₅₀-Wert im Bereich von 100 bis 5000 µm zur Anwendung.

Das unreagierte stöchiometrische Gemisch der Ausgangsstoffe zur Synthetisierung des Calciumphosphates kommt zur Verstärkung der Porengrößenverteilung (I) in einem Mengenanteil von 1 bis 50 Masse-% zum Einsatz. Während des Reaktions-/Sinterbrandes setzt es sich zum gewünschten Calciumphosphat um, ohne dessen Reinheit zu beeinträchtigen. Es verstärkt den Anteil an Poren im Bereich der Porengrößenverteilung (I) und führt gleichzeitig zu einer Verfestigung des Sinterstruktur des Calciumphoosphates.

Ausbrennbares Porosierungsmittel zur Herstellung des erfindungsgemäßen Knochenaufbaumittels ist Ammoniumhydrogencarbonat. Zur Verstärkung einer Porengrößenverteilung (I) wird dieses der Mischung der Calciumphosphatbestandteile als Kornfraktion mit einem d₅₀-Wert im Bereich von 0,5 bis 10 µm in einer Menge von 1 bis 20 Masse-% zugesetzt. Für die Erzeugung der Porengrößenverteilung (II) erfolgt der Zusatz des Ammoniumhydrogencarbonates mit einem d₅₀-Wert im Bereich von 10 bis 100 µm in einer Menge von 5 bis 40 Masse-%, während für die Erzeugung der Porengrößenverteilung (III) dessen Kornfraktion mit einem d₅₀-Wert im Bereich von 100 bis 5000 µm mit einer Menge von 1 bis 40 Masse-% zugesetzt wird. Die verwendeten Mengen an Porosierungsmittel sind auf die Einsatzmenge an Calciumphosphat berechnet.

Neben einer intensiven Homogenisierung der Mischung der Calciumphosphatbestandteile und deren Homogenisierung ohne weitere Kornzerkeinerung mit dem ausbrennbaren Porosierungsmittel kommt vor allem bei der Herstellung vom Rohteilen für eine maschinelle Bearbeitung der Kompaktierung eine entscheidende Bedeutung zu. Hier haben sich im Rahmen der experimentellen Untersuchungen an Standardproben das isostatische Pressverfahren mit Pressdrücken im Bereich von 100 bis 250 MPa als vorteilhaft erwiesen.

Die kompaktierte Mischung der Calciumphosphatbestandteile und Porosierungsmittel wird einer kontrollierten Wärmebehandlung unterworfen, ggf. auch in mehreren Behandlungsschritten, bei der die Porosierungsmittel absublimieren oder ausbrennen, die unumgesetzten Ausgangsstoffe in ihrem stöchiometrischen Verhältnis zum gewünschten Calciumphosphat reagieren, die eingesetzten Pulverpartikel des Calciumphosphates praktisch "verkitten" und feste Sinterhälse erzeugen. Die Anwesenheit der Pulverpartikel des gewünschten Calciumphosphates begünstigt gleichzeitig die Bildung dieser Phase aus den unreagierten Ausgangsrohstoffen durch entsprechende Keimbildung und Kristallisation, so dass eine hohe Phasenreinheit des gebildeten Calciumphosphates größer 99 Masse-% erzielt wird.

Die Wärmebehandlung der kompaktierten Mischung erfolgt sowohl durch kontrollierte Aufheiz- und Abkühlprogramme, verbunden mit Haltestufen in relevanten Temperaturbereichen. Als Aufheiz- und Abkühlgeschwindigkeiten haben sich Werte im Bereich von 0,5 bis 5 K/min als vorteilhaft erwiesen. Je kompakter und massiver die Sinterteile sind, desto geringer wird die verwendete Aufheiz- bzw. Abkühlgeschwindigkeit gewählt. Geeignete Haltetemperaturen für eine mechanisch anspruchsvolle Sinterstruktur liegen im Bereich von 1373 bis 1573 K, wobei sich die gewählte Höhe der Sintertemperatur nach der zugesetzten Menge der stöchiometrischen Mischung der unumgesetzten Ausgangsstoffe richtet. Mit zunehmender Menge dieser Mischung kann die Sintertemperatur zu niedrigeren Werten verschoben werden, um vergleichbare mechanische Festigkeiten der Sinterkörper zu realisieren. Im speziellen Falle der Herstellung von Beta-Tricalciumphosphat kann es im Sinne der Phasenreinheit auch vorteilhaft sein, im Bereich von 1123 bis 1223 K eine weitere Haltestufe einzulegen, um Phasenanteile von Alpha-Tricalciumphosphat eindeutig auszuschließen.

Gemäß einer weiteren Ausführungsform betrifft die Erfindung ein Knochenaufbaumittel aus Calciumphosphat mit einer aus mehreren Porengrößenverteilungsbereiche zusammengesetzten Gesamtporosität, dadurch gekennzeichnet, dass es eine in seiner geometrischen Gestalt irreguläre Gesamtporosität aus drei diskreten Bereichen von in ihrer Größe statistisch verteilten Porengrößen aufweist, dass das Calciumphosphat eine Primärkorngröße kleiner 63 µm mit einem d₅₀-Wert im Bereich von 5 bis 20 µm aufweist und dass der interkonnektierende Porenanteil an der Gesamtporosität auf Porengrößen kleiner 10 µm begrenzt ist.

Das Knochenaufbaumittel ist dadurch gekennzeichnet, dass die Maxima der drei diskreten Porengrößenverteilungsbereiche in den Durchmesserbereichen 0,5 bis 10 µm (I), 10 bis 100 µm (II) und 100 bis 5000 µm (III) liegen.

Ferner kann das Knochenaufbaumittel dadurch gekennzeichnet sein, dass die Volumenverhältnisse der drei diskreten Porengrößenverteilungen für die Porengrößenverteilung (I) im Bereich von 20 bis 40 Vol.-%, für die Porengrößenverteilung (II) im Bereich von 5 bis 40 Vol.-% und für die Porengrößenverteilung (III) im Bereich von 1 und insbesondere 5 bis 40 Vol.-% liegen, wobei die Gesamtporosität einen Betrag von 80 und insbesondere 85 Vol.-% nicht überschreitet.

Ferner kann das Knochenaufbaumittel dadurch gekennzeichnet sein, dass das Calciumphosphat im wesentlichen und insbesondere zu wenigstens 95 % aus Alpha-Tricalciumphosphat, Beta-Tricalciumphosphat, Octacalciumphosphat, alkali- und/oder erdalkalimodifiziertem Tricalciumphosphat, Calciumdiphosphat, Carbonatapatit vom B-Typ, Calcium-defizienten Hydroxylapatit oder deren Gemischen besteht.

Ferner kann das Knochenaufbaumittel dadurch gekennzeichnet sein, dass das Calciumphosphat vorzugsweise aus Beta-Tricalciumphosphat mit einer Phasenreinheit ≥ 99 Masse-%, bezogen auf die Fremdphase Hydroxylapatit besteht.

Ferner kann das Knochenaufbaumittel dadurch gekennzeichnet sein, dass es als Granulat ausgebildet ist und in verschiedenen, indikationsbezogenen Granulatfraktionen in einem Größenbereich zwischen 50 und 10000 µm vorliegt.

Ferner kann das Knochenaufbaumittel dadurch gekennzeichnet sein, dass das Granulat eine im wesentlichen uneinheitliche geometrische Gestalt aufweist.

Ferner kann das Knochenaufbaumittel dadurch gekennzeichnet sein, dass das Granulat eine im wesentlichen einheitliche geometrische Gestalt aufweist.

Ferner kann das Knochenaufbaumittel dadurch gekennzeichnet sein, dass das Granulat eine im wesentlichen kugelige Gestalt aufweist.

Ferner kann das Knochenaufbaumittel dadurch gekennzeichnet sein, dass die Porengrößenverteilungen (III) auf die Granulatgröße abgestimmt sind, wobei die mittlere Porengröße einen Betrag kleiner als die Hälfte der mittleren Granulatgröße der jeweiligen Granulatfraktion aufweist und vorzugsweise in einem Bereich der mittleren Granulatgröße von 10 bis 50 % liegt.

Ferner kann das Knochenaufbaumittel dadurch gekennzeichnet sein, dass es als Formkörper mit einem definierten geometrischen Design ausgebildet ist.

Ferner kann das Knochenaufbaumittel dadurch gekennzeichnet sein, daß es zusätzlich zu einer statistischen Porosität eine.gerichtete Porosität in Form von Röhrenporen aufweist, insbesondere vom Typ (III).

Ferner kann das Knochenaufbaumittel dadurch gekennzeichnet sein, dass die gerichtete Röhrenporosität durch maschinell eingebrachte ein-, zwei- oder dreidimensionaler Bohrungen im Durchmesserbereich von 0,5 bis 2 mm ausgebildet ist und die Gesamtporosität aus statistischer und Röhrenporosität einen Wert von 85 Vol.-% nicht übersteigt.

Ferner kann das Knochenaufbaumittel dadurch gekennzeichnet sein, dass der kompakte Formkörper eine in Größe und Form abgestufte Porengrößenverteilung vom Rand zum Kern hin aufweist, wobei vorzugsweise in der Randzone die Porengrößenverteilungen (I) und/oder (II), insbesondere mit einer Gesamtporosität bis zu 35 Vol.-%, in der Kernzone die Porengrößenverteilungen (I) und/oder (II) und/oder (III), insbesondere bis zu einer Gesamtporosität von 85 Vol.-%, vorliegen, wobei insbesondere die Randzone einen Bereich von 10 % bis 40 % und die Kernzone von 60 % bis 90 % des Implantatgrößtmaßes senkrecht zur Zugspannungsrichtung bzw. parallel zur Biegebeanspruchung aufweist.

Ferner kann das Knochenaufbaumittel dadurch gekennzeichnet sein, dass es auf seiner Oberfläche und/oder in seiner inneren Porenstruktur antibakterielle, wundheilungsfördernde, knochenwachstumsfördernde und/oder gerinnungshemmende Substanzen in geeigneter, wirksamer Konzentration aufweist.

Ferner kann das Knochenaufbaumittel dadurch gekennzeichnet sein, dass es eine individuell für einen bestimmten Patienten angefertigte Form aufweist.

Ferner kann das Knochenaufbaumittel dadurch gekennzeichnet sein, dass es in standardisierte Abmessungen und Formen, vorzugsweise als Würfel, Quader, Zylinder oder Keil vorliegt.

Ferner kann das Knochenaufbaumittel dadurch gekennzeichnet sein, dass es eine indikationsbezogene Form, vorzugsweise als Trepanationsverschluss, Alveolaraugmentat oder Füllkörper für Cages zum Wirbelkörperersatz aufweist.

Gemäß einer weiteren Ausführungsform betrifft die Erfindung ein Verfahren zur Herstellung eines aus Calciumphosphat bestehenden Knochenaufbaumittels über den Syntheseweg einer thermisch induzierten Festkörperreaktion aus einem stöchiometrischen Gemisch zweier vorzugswise bekannter Ausgangsstoffe (1, 2), deren homogener Vermischung, Sinterung und Zerkleinerung sowie nachfolgender Zumischung von ausbrennbaren oder sich verflüchtigenden Porosierungsmitteln, das dadurch gekennzeichnet ist, dass dem aus den Ausgangsstoffen (1, 2) synthetisierten Calciumphosphat (C) nach Herstellung und Zerkleinerung zur Erzeugung einer Mikroporosität ein weiterer Anteil des unumgesetzten, stöchiometrischen Gemisches der Ausgangsstoffe (1, 2) und drei weitere, ausbrennbare Porosierungsmittel zur Verstärkung des Porenanteils (I) sowie zur Erzeugung der Porenanteile (II) und (III) gemäß Anspruch 2 zugesetzt werden, die Mischung homogenisiert, kompaktiert und zu einem porösen Sinterkörper gebrannt wird.

Das Verfahren kann dadurch gekennzeichnet sein, dass dem aus den Ausgangsstoffen (1, 2) hergestellten Calcium-phosphat (C) das unumgesetzte, stöchiometrische Gemisch der Ausgangsstoffe (1, 2) in einer Menge zwischen 1 und 50 Masse-%, bezogen auf die Calciumphosphatmenge, zugesetzt wird.

Ferner kann das Verfahren dadurch gekennzeichnet sein, dass es sich bei dem Calciumphosphat (C) um Tricalciumphosphat, vorzugsweise ≥ 99 Masse-% phasenreines Beta-Tricalciumphosphat und bei den Ausgangsstoffen (1, 2) um Calciumcarbonat und Calciumhydrogenphosphat handelt.

Ferner ist das Verfahren dadurch gekennzeichnet, dass das in die Mischung eingebrachte Calciumphosphat eine Primärkorngröße kleiner 63 µm bei einem d₅₀-Wert im Bereich von 5 bis 20 µm aufweist.

Ferner ist das Verfahren dadurch gekennzeichnet, dass die zugesetzten, ausbrennbaren oder sich verflüchtigenden Porosierungsmittel in Kornfraktionen mit d₅₀-Werten im Bereich von 0,5 bis 10 µm, 10 bis 100 µm und 100 bis 5000 µm zugesetzt werden.

Ferner ist das Verfahren dadurch gekennzeichnet, dass der Calciumphosphatmischung bei Verwendung des Porosierungsmittels Ammoniumhydrogencarbonat die Kornfraktion mit einen d₅₀-Wert im Bereich von 0,5 bis 10 µm in einer Menge von 1 bis 20 Masse-%, die Kornfraktion mit einem d₅₀-Wert im Bereich von 10 bis 100 µm in einer Menge von 5 bis 40 Masse-% und die Kornfraktion mit einen d₅₀-Wert im Bereich von 100 bis 5000 µm mit einer Menge im Bereich von 1 bis 40 Masse-%, bezogen auf die berechnete Menge Calciumphosphat, zugesetzt wird.

Ferner kann das Verfahren dadurch gekennzeichnet sein, dass die Kompaktierung der Mischung des Calciumphosphates (C), des Anteils des unumgesetzten, stöchiometrischen Gemisches der Ausgangsstoffe (1, 2) sowie der Porosierungsmittel isostatisch bei einem Pressdruck von 100 bis 250 MPa erfolgt.

Ferner kann das Verfahren dadurch gekennzeichnet sein, dass die kompaktierte Mischung des/der Calciumphosphate(s) (C), der stöchiometrischen Mischung der unreagierten Ausgangsstoffe (1, 2) sowie des/der ausbrennbaren Porosierungsmittel(s) mit einer Aufheizgeschwindigkeit im Bereich von 0,5 bis 5 K/min in den Bereich von 1373 bis 1573 K erhitzt, bei dieser Temperatur vorzugsweise 24 bis 72 Stunden gehalten und anschließend mit einer Abkühlgeschwindigkeit von 0,5 bis 5 K/min wieder auf Raumtemperatur abgekühlt wird.

Ferner kann das Verfahren dadurch gekennzeichnet sein, dass bei der kontrollierten Temperaturbehandlung eine zusätzliche Temperatur-Haltestufe im Bereich von 1123 bis 1223 K verwendet wird.

**Figur 1** zeigt schematisch ein Beispiel für ein erfindungsgemäßes Knochenaufbaumittel mit drei Porengrößenverteilungen (I), (II) und (III).

**Figur 2** **und** **3** skizzieren die Herstellung eines Gradientenwerkstoffes mit von außen nach innen abnehmender Dichte resp. zunehmender Porosität gemäß Ausführungsbeispiel 8.

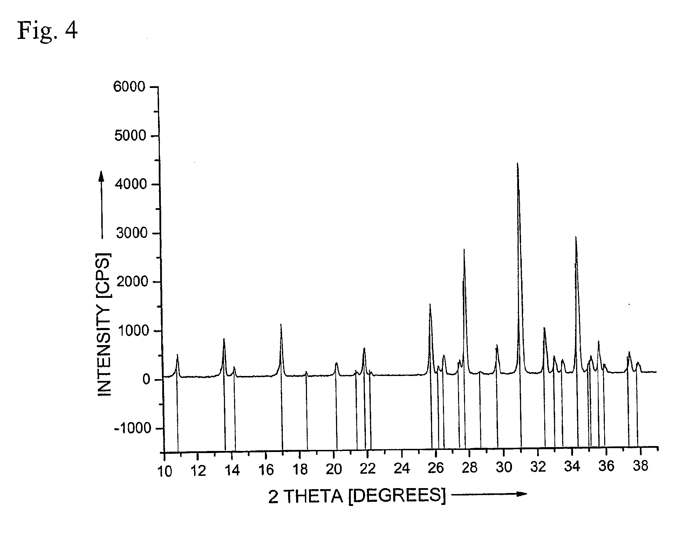

**Figur 4** belegt die Phasenreinheit des verwendeten Beta-Tricalciumphosphates mit größer 99 Masse-% bezogen aus die Phase Hydroxylapatit.

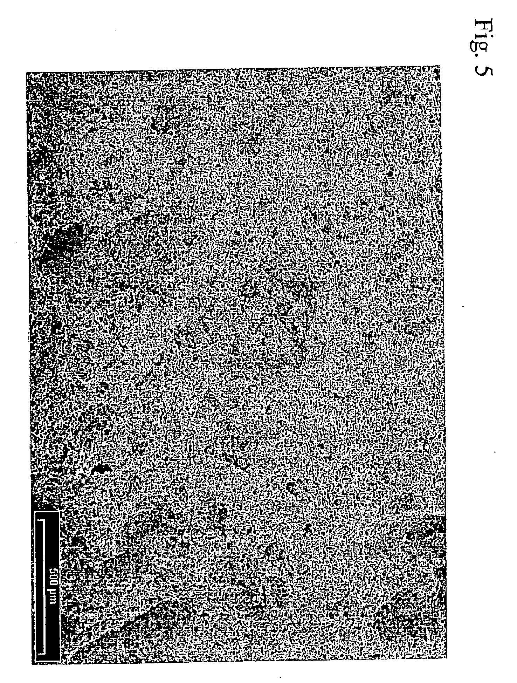

**Figur 5** veranschaulicht die geschlossenzellige Sinterstruktur eines Granulatkornes an einer frischen Bruchfläche mittel REM

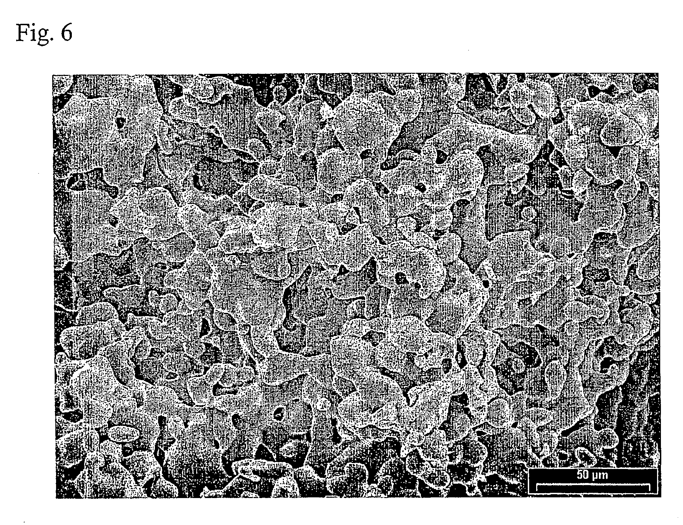

**Figur 6** zeigt das interkonnektierende Mikroporensystem kleiner 10 µm mit einzelnen über dieses Netzwerk verbundenen Poren der Größe 30 bis 50 µm an einer frischen Bruchfläche eines Granulatkornes

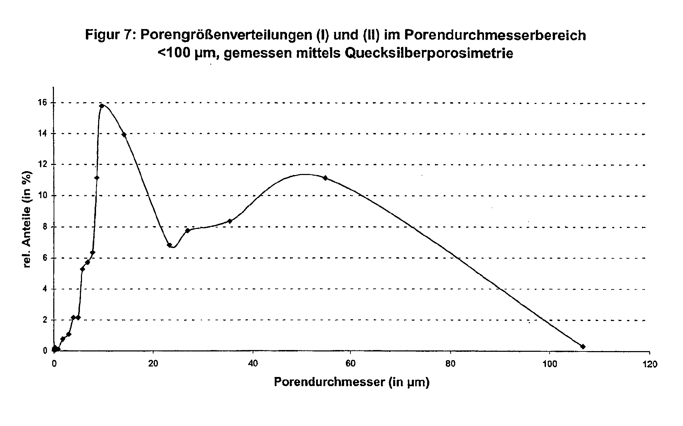

**Figur 7** veranschaulicht eine typische Kurve der Porengrößenverteilungen (I) und (II) mit zwei Maxima im Bereich < 100 µm, gemessen mittels Quecksilberporosimetrie.

Nachfolgend wird die Erfindung an ausgewählten Ausführungsbeispielen beschrieben. Für die Herstellung von beispielsweise porosiertem Beta-Tricalciumphosphat werden die Ausgangsstoffe (A) bis (F) bereitgestellt:
**Ausgangsstoff (A):** Calciumcarbonat, CaCO₃ p.a. in Pulverform, trocken
**Ausgangsstoff (B):** Calciumhydrogenphosphat , CaHPO₄, p.a., in Pulverform, trocken
**Ausgangstoff (C):** Phasenreines Beta-Tricalciumphosphat, Ca, Ca₃(PO₄)₂, in Pulverform, trocken, kleiner 63 µm (d₅₀ gleich 12 µm)
**Ausgangsstoff (D):** Ammoniumhydrogencarbonat p.a. mit einem d₅₀ - Wert im Bereich von 0,5 bis 10 µm
**Ausgangsstoff (E):** Ammoniumhydrogencarbonat p.a. mit einem d₅₀ - Wert im Bereich von 10 bis 100 µm
**Ausgangsstoff (F):** Ammoniumhydrogencarbonat p.a. mit einem d₅₀ - Wert im Bereich von 100 bis 5000 µm

### Ausführungsbeispiel 0:

Zur Herstellung des Ausgangsstoffes (C) werden die Ausgangsstoffe (A) und (B) im Molverhältnis 1:2 homogen vermischt. Die Mischung wird mittels eines Druckes von 150 MPa kompaktiert und über eine Zeitdauer von 20 Stunden bei 1200°C unter Normalatmosphäre erhitzt. Das entstandene Reaktionsprodukt Beta-Tricalciumphosphat weist eine Phasenreinheit von > 99% auf, wird auf eine Korngröße < 63 µm zerkleinert und fraktioniert und steht dann als Ausgangsstoff (C) für die weiteren Arbeiten zur Verfügung.

### Ausführungsbeispiel 1:

Dem Ausgangsstoff (C) werden 10 Masse-% einer Mischung der Ausgangsstoffe (A) und (B) im Molverhältnis 1:2 zugesetzt und alle Bestandteile innig vermischt. Nachfolgend wird der Mischung 10 Masse-% des Ausgangsstoffs (D) mit einem d₅₀ - Wert von 8 µm, 35 Masse-% des Ausgangsstoffs (E) mit einem d₅₀ - Wert von 35 µm und 5 Masse-% des Ausgangsstoffs (F) mit einem d₅₀ - Wert von 350 µm zugegeben und vermengt.

Die Mischung wird mittels eines Druckes von 150 MPa kompaktiert und die Ausgangsstoffe (D), (E) und (F) über 20 Stunden bei 80°C an Normalatmosphäre absublimiert. Danach wird das kompaktierte, poröse Material bei 1200°C über 20 Stunden an Normalatmosphäre gesintert und anschließend in eine Kornfraktion von 500-1000 µm gebrochen. Das Granulat wird zur Abrundung der Ecken mit sich selbst in einer PE-Flasche auf einem Walzenstuhl mit einer Umdrehungsgeschwindigkeit von 30 U/min umgewälzt und anschließend in einzelne Kornfraktionen klassiert.

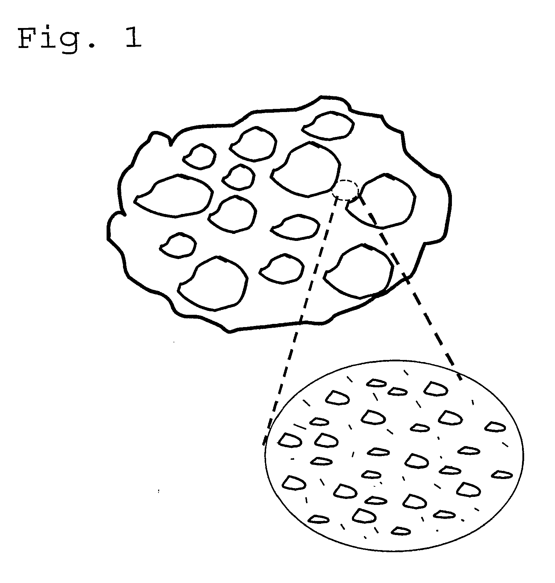

Die entstandenen Granulatkörner besitzen eine Gesamtporosität von 72%. Die Schüttdichten der nach diesem Verfahren entstandenen Granulatkörner betragen 0,9 g/cm³. Die Schüttdichte von kompaktiertem Material ohne zusätzliche Porosierungsmittel beträgt durchschnittlich 1,2 g/cm³. Die Granulatkörner weisen eine Porenstruktur mit drei diskreten Porengrößenverteilungen gemäß Figur 1 auf.

### Ausführungsbeispiel 2:

Dem Ausgangsstoff (C) werden 20 Masse-% einer Mischung der Ausgangsstoffe (A) und (B) im Molverhältnis 1:2 zugesetzt und alle Bestandteile innig vermischt. Nachfolgend wird der Mischung 20 Masse-% des Ausgangsstoffs (D) mit einem d₅₀ - Wert von 8 µm, 25 Masse-% des Ausgangsstoffs (E) mit einem d₅₀ - Wert von 35 µm und 10 Masse-% des Ausgangsstoffs (F) mit einem d₅₀ - Wert von 250 µm zugegeben und vermengt.

Die Mischungwird mittels eines Druckes von 170 MPa kompaktiert und die Ausgangsstoffe (D), (E) und (F) über 20 Stunden bei 80°C an Normalatmosphäre absublimiert. Danach wird das kompaktierte, poröse Material bei 1200°C über 20 Stunden an Normalatmosphäre gesintert und anschließend in eine Kornfraktion von 500-1000 µm gebrochen. Das Granulat wird zur Abrundung der Ecken mit sich selbst in einer PE-Flasche auf einem Walzenstuhl mit einer Umdrehungsgeschwindigkeit von 30 U/min umgewälzt und anschließend in einzelne Kornfraktionen klassiert.

Die entstandenen Granulatkörner besitzen eine Gesamtporosität von 78%. Die Schüttdichten der nach diesem Verfahren entstandenen Granulatkörner betragen 0,8 g/cm³. Die Schüttdichte von kompaktiertem Material ohne zusätzliche Porosierungsmittel beträgt durchschnittlich 1,2 g/cm³. Die Granulatkörner weisen eine Porenstruktur mit drei diskreten Porengrößenverteilungen gemäß Figur 1 auf.

### Ausführungsbeispiel 3:

Dem Ausgangsstoff (C) werden 30 Masse-% einer Mischung der Ausgangsstoffe (A) und (B) im Molverhältnis 1:2 zugesetzt und alle Bestandteile innig vermischt. Nachfolgend wird der Mischung 20 Masse-% des Ausgangsstoffs (D) mit einem d₅₀ - Wert von 6,5 µm, 5 Masse-% des Ausgangsstoffs (E) mit einem d₅₀ - Wert von 65 µm und 20 Masse-% des Ausgangsstoffs (F) mit einem d₅₀ - Wert von 650 µm zugegeben und vermengt.

Die Mischung wird mittels eines Druckes von 170 MPa kompaktiert und die Ausgangsstoffe (D), (E) und (F) über 20 stunden bei 80°C an Normalatmosphäre absublimiert. Danach wird das kompaktierte, poröse Material bei 1200°C über 20 Stunden an Normalatmosphäre gesintert und anschließend in eine Kornfraktion von 1000-2000 µm gebrochen. Das Granulat wird zur Abrundung der Ecken mit sich selbst in einer PE-Flasche auf einem Walzenstuhl mit einer Umdrehungsgeschwindigkeit von 30 U/min umgewälzt und anschließend in einzelne Kornfraktionen klassiert.

Die entstandenen Granulatkörner besitzen eine Gesamtporosität von 70%. Die Schüttdichten der nach diesem Verfahren entstandenen Granulatkörner betragen 0,9 g/cm³. Die Schüttdichte von kompaktiertem Material ohne zusätzliche Porosierungsmittel beträgt durchschnittlich 1,2 g/cm³. Die Granulatkörner weisen eine Porenstruktur mit drei diskreten Porengrößenverteilungen gemäß Figur 1 auf.

### Ausführungsbeispiel 4:

Dem Ausgangsstoff (C) werden 40 Masse-% einer Mischung der Ausgangsstoffe (A) und (B) im Molverhältnis 1:2 zugesetzt und alle Bestandteile innig vermischt. Nachfolgend werden der Mischung 5 Masse-% des Ausgangsstoffs (D) mit einem d₅₀ - Wert von 5 µm, 15 Masse-% des Ausgangsstoffs (E) mit einem d₅₀ - Wert von 65 µm und 35 Masse-% des Ausgangsstoffs (F) mit einem d₅₀ - Wert von 650 µm zugegeben und vermengt.

Die Mischung wird mittels eines Druckes von 180 MPa kompaktiert und die Ausgangsstoffe (D), (E) und (F) über 20 Stunden bei 80°C an Normalatmosphäre absublimiert. Danach wird das kompaktierte, poröse Material bei 1200°C über 20 Stunden an Normalatmosphäre gesintert und anschließend in eine Kornfraktion von 500-1000 µm gebrochen. Das Granulat wird zur Abrundung der Ecken mit sich selbst in einer PE-Flasche auf einem Walzenstuhl mit einer Umdrehungsgeschwindigkeit von 30 U/min umgewälzt und anschließend in einzelne Kornfraktionen klassiert.

Die entstandenen Granulatkörner besitzen eine Gesamtporosität von 81%. Die Schüttdichten der nach diesem Verfahren entstandenen Granulatkörner betragen 0,8 g/cm³. Die Schüttdichte von kompaktiertem Material ohne zusätzliche Porosierungsmittel beträgt durchschnittlich 1,2 g/cm³. Die Granulatkörner weisen eine Porenstruktur mit drei diskreten Porengrößenverteilungen gemäß Figur 1 auf.

### Ausführungsbeispiel 5:

Dem Ausgangsstoff (C) werden 25 Masse-% einer Mischung der Ausgangsstoffe (A) und (B) im Molverhältnis 1:2 zugesetzt und alle Bestandteile innig vermischt. Nachfolgend werden der Mischung 15 Masse-% des Ausgangsstoffs (D) mit einem d₅₀ - Wert von 5 µm, 15 Masse-% des Ausgangsstoffs (E) mit einem d₅₀ - Wert von 85 µm und 15 Masse-% des Ausgangsstoffs (F) mit einem d₅₀ - Wert von 1850 µm zugegeben und innig vermengt.

Die Mischung wird mittels eines Druckes von 190 MPa kompaktiert und die Ausgangsstoffe (D), (E) und (F) über 20 Stunden bei 80°C an Normalatmosphäre absublimiert. Danach wird das kompaktierte, poröse Material bei 1200°C über 20 Stunden an Normalatmosphäre gesintert und anschließend in eine Kornfraktion von 3200-5000 µm gebrochen. Das Granulat wird zur Abrundung der Ecken mit sich selbst in einer PE-Flasche auf einem Walzenstuhl mit einer Umdrehungsgeschwindigkeit von 30 U/min umgewälzt und anschließend in einzelne Kornfraktionen klassiert.

Die entstandenen Granulatkörner besitzen eine Gesamtporosität von 69%. Die Schüttdichten der nach diesem Verfahren entstandenen Granulatkörner betragen 0,9 g/cm³. Die Schüttdichte von kompaktiertem Material ohne zusätzliche Porosierungsmittel beträgt durchschnittlich 1,2 g/cm³. Die Granulatkörner weisen eine Porenstruktur mit drei diskreten Porengrößenverteilungen gemäß Figur 1 auf.

### Ausführungsbeispiel 6:

Dem Ausgangsstoff (C) werden 20 Masse-% einer Mischung der Ausgangsstoffe (A) und (B) im Molverhältnis 1:2 zugesetzt und alle Bestandteile innig vermischt. Nachfolgend werden der Mischung 20 Masse-% des Ausgangsstoffs (D) mit einem d₅₀ - Wert von 5 µm, 20 Masse-% des Ausgangsstoffs (E) mit einem d₅₀ - Wert von 65 µm und 10 Masse-% des Ausgangsstoffs (F) mit einem d₅₀ - Wert von 250 µm zugegeben und innig vermengt.

Die Mischung wird mittels eines Druckes von 200 MPa kompaktiert und die Ausgangsstoffe (D), (E) und (F) über 20 Stunden bei 80°C an Normalatmosphäre absublimiert. Danach wird das kompaktierte, poröse Material bei 1200°C über 20 Stunden an Normalatmosphäre gesintert. Die so entstandenen porösen Grundkörper werden mechanisch zu Zylindern, Quadern und Würfeln bearbeitet.

Die Dichte des Keramikmaterials beträgt vor dem Absublimieren der Ausgangsstoffe (D), (E) und (F) durchschnittlich 1,6 g/cm³, danach durchschnittlich 0,8 g/cm³. Die Gesamtporosität lag bei 73%. Die Formkörper weisen drei diskrete Porengrößenverteilungen gemäß Figur 1 auf.

### Ausführungsbeispiel 7:

Dem Ausgangsstoff (C) werden 20 Masse-% einer Mischung der Ausgangsstoffe (A) und (B) im Molverhältnis 1:2 zugesetzt und alle Bestandteile innig vermischt. Nachfolgend werden der Mischung 25 Masse-% des Ausgangsstoffs (D) mit einem d₅₀ - Wert von 8 µm, 20 Masse-% des Ausgangsstoffs (E) mit einem d₅₀ - Wert von 35 µm und 15 Masse-% des Ausgangsstoffs (F) mit einem d₅₀ - Wert von 350 µm zugegeben und innig vermengt.

Die Mischung wird mittels eines Druckes von 200 MPa kompaktiert und die Ausgangsstoffe (D), (E) und (F) über 20 Stunden bei 80°C an Normalatmosphäre absublimiert. Danach wird das kompaktierte, poröse Material bei 1200°C über 20 Stunden an Normalatmosphäre gesintert. Die so entstandenen porösen Grundkörper werden mechanisch zu Keilen, Trepanationsverschlüssen und Alveolaraugmentaten bearbeitet.

Die Dichte des Keramikmaterials beträgt vor dem Absublimieren der Ausgangsstoffe (D), (E) und (F) durchschnittlich 1,6 g/cm³, danach durchschnittlich 0,6 g/cm³. Die Gesamtporosität lag bei 83%. Die Formkörper weisen drei diskrete Porengrößenverteilungen gemäß Figur 1 auf.

### Ausführungsbeispiel 8:

Dem Ausgangsstoff (C) werden 30 Masse-% einer Mischung der Ausgangsstoffe (A) und (B) im Molverhältnis 1:2 zugesetzt und alle Bestandteile innig vermischt. Nachfolgend werden der Mischung 15 Masse-% des Ausgangsstoffs (D) mit einem d₅₀ - Wert von 4 µm, 5 Masse-% des Ausgangsstoffs (E) mit einem d₅₀ - Wert von 85 µm und 5 Masse-% des Ausgangsstoffs (F) mit einem d₅₀ - Wert von 250 µm zugegeben und innig vermengt.

Die Mischung wird mittels eines Druckes von 250 MPa kompaktiert und die Ausgangsstoffe (D), (E) und (F) über 20 Stunden bei 80°C an Normalatmosphäre absublimiert. Danach wird das kompaktierte, poröse Material bei 1200°C über 20 Stunden an Normalatmosphäre gesintert. Die so entstandenen porösen Grundkörper werden mechanisch zu Zylindern, Quadern und Würfelns bearbeitet und mit einem dreidimensionalen Bohrmuster mit einem Bohrlochdurchmesser von 1 mm versehen.

Die Dichte des Keramikmaterials beträgt vor dem Absublimieren der Ausgangsstoffe D, E und F durchschnittlich 1,6 g/cm³, danach durchschnittlich 1,4 g/cm³. Nach dem Einbringen der gerichteten Röhrenporosität lag die Gesamtporosität aus statistischer und gerichteter Porosität bei 75%. Die Formkörper weisen neben der gerichteten Röhrenporosität drei diskrete Porengrößenverteilungen gemäß Figur 1 auf.

### Ausführungsbeispiel 9:

Dem Ausgangsstoff (C) werden 30 Masse-% einer Mischung der Ausgangsstoffe (A) und (B) im Molverhältnis 1:2 zugesetzt und alle Bestandteile innig vermischt.

Die Mischung wird in 3 Unterportionen unterteilt. Der Unterportion (1) werden 5 Masse-% des Ausgangsstoffs (D) mit einem d₅₀ - Wert von 4 µm und 10 Masse-% des Ausgangsstoffs (E) mit einem d₅₀ - Wert von 35 µm zugesetzt, der Unterportion (2) 10 Masse-% des Ausgangsstoffes (D) mit einem d₅₀ - Wert von 6,5 µm und 20 Masse-% des Ausgangsstoffes (E) mit einem d₅₀ - Wert von 65 µm und der Unterportion (3) werden 20 Masse-% des Ausgangsstoffes (D) mit einem d₅₀ - Wert von 8 µm, 20 Masse-% des Ausgangsstoffes (E) mit einem d₅₀ - Wert von 85 µm und 20 Masse-% des Ausgangsstoffs (F) mit einem d₅₀ - Wert von 650 µm zugegeben und die Unterportionen einzeln innig vermengt.

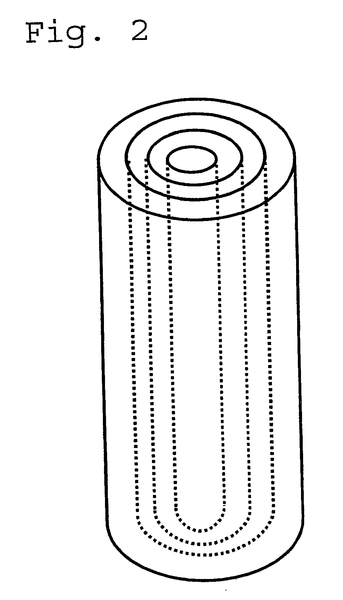

Eine flexible Pressform für Kalt-isostatisches Pressen wird mit zwei ineinander gestellte Röhren versehen, die den gewünschten Zwischenabstand aufweisen (siehe Fig. 2). In diese Zwischenräume werden die Unterportionen eingefüllt und zwar so, dass von Innen nach Außen die Menge der zugefügten Ausgangsstoffe (D), (E) und (F) abnehmen. Nach erfolgtem Einfüllen werden die Röhren vorsichtig entfernt, so dass nur eine oberflächliche Pulververmischung der einzelnen Unterportionen stattfindet.

Das Material wird in der flexiblen Pressform mittels eines Drukkes von 200 MPa kompaktiert und die Ausgangsstoffe (D), (E) und (F) über 20 Stunden bei 80°C an Normalatmosphäre absublimiert. Danach wird das kompaktierte, poröse Material bei 1200°C über 20 Stunden an Normalatmosphäre gesintert. Die so entstandenen porösen Körper werden mechanisch bearbeitet um Formkörper gemäß der Ausführungsbeispiele 6 bis 8 zu gewinnen.

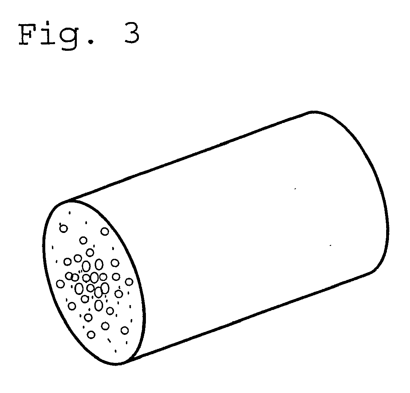

Es entsteht ein Gradientenwerkstoff, dessen Porosität von außen nach innen zunimmt. Dadurch ist eine erhöhte mechanische Belastbarkeit einstellbar, ebenso wie lokal unterschiedliche Resorptionsgeschwindigkeiten (siehe Fig. 3).

### Ausführungsbeispiel 10:

Bei einem zylindrischen Teil, hergestellt gemäß Ausführungsbeispiel 9, wurden zusätzlich in der Randzone geringer Porosität in Knochenwachstumsrichtung Röhrenporen mit einem Durchmesser von 1,4 mm eingebracht. Dadurch wird der dichte Materialbereich schneller knöchern erschlossen, ohne die guten mechanischen Eigenschaften der Randzone zu schmälern.

## Patentansprüche

1. Knochenaufbaumittel aus porösem Calciumphosphat mit isotroper Sinterstruktur und statistisch zwischen den versinterten Partikeln des Calciumphosphates verteilten Poren in mehreren diskreten Größenverteilungen, **dadurch gekennzeichnet, dass** es eine aus drei diskreten Porengrößenverteilungen (I), (II) und (III) zusammengesetzte Porosität aufweist und die Maxima der drei diskreten Porengrößenverteilungen bei Porendurchmessern in den Intervallen 0,5 bis 10 µm (I), 10 bis 100 µm (II) und 100 bis 5000 µm (III) liegen, die Porosität eine irreguläre geometrische Gestalt besitzt, die versinterten Partikel des Calciumphosphates eine Partikelgröße kleiner 63 µm mit einem d₅₀-Wert im Bereich von 5 bis 20 µm aufweisen und der interkonnektierende Porenanteil der Porosität auf Porengrößen kleiner 10 µm begrenzt ist; dadurch erhältlich, dass ein Calciumphosphat als Ausgangsstoff (C) aus seinen Ausgangsrohstoffen (A) und (B) synthetisiert wird, nachfolgend der synthetisierte Ausgangsstoff (C) auf eine Korngröße kleiner 63 µm mit einem d₅₀-Wert im Bereich von 5 bis 20 µm zerkleinert wird und mit einem Anteil seiner unumgesetzten Ausgangsstoffe (A) und (B) im stöchiometrischen Verhältnis versetzt wird, das Gemisch intensiv homogenisiert wird, nachfolgend das ausbrennbare oder sich verflüchtigende Porosierungsmittel Ammoniumhydrogencarbonat in Kornfraktionen mit d₅₀-Werten im Bereich von 0,5 bis 10 µm, 10 bis 100 µm und 100 bis 5000 µm zugesetzt werden, wobei die Kornfraktion des Porosierungsmittels mit einem d₅₀-Wert im Bereich von 0,5 bis 10 µm in einer Menge von 1 bis 20 Masse-%, die Kornfraktion des Porosierungsmittels mit einem d₅₀-Wert im Bereich von 10 bis 100 µm in einer Menge von 5 bis 40 Masse-% und die Kornfraktion Porosierungsmittels mit einem d₅₀-Wert im Bereich von 100 bis 5000 µm mit einer Menge im Bereich von 1 bis 40 Masse-%, jeweils bezogen auf die berechnete Menge Calciumphosphat, zugesetzt wird; die Calciumphosphatbestandteile mit den Fraktionen der ausbrennbaren Porosierungsmittel ohne weitere Zerkleinerung homogenisiert und nachfolgend kompaktiert werden; die Porosierungsmittel durch Erhitzen entfernt werden; die porösen, ungebrannten Calciumphosphatkörper auf Reaktions-/Sintertemperatur für eine erforderliche Zeit erhitzt werden; die porösen Sinterkörper nachfolgend auf Raumtemperatur abgekühlt und in die gewünschte Form als Granulat oder Formteil gebracht werden.

2. Knochenaufbaumittel nach Anspruch 1, **dadurch gekennzeichnet, dass** die Volumenanteile der diskreten Porengrößenverteilungen (I) bis (III) für die Porengrößenverteilung (I) im Bereich von 20 bis 40 Vol.-%, für die Porengrößenverteilung (II) im Bereich von 5 bis 40 Vol.-% und für die Porengrößenverteilung (III) im Bereich von 1 bis 40 Vol.-% liegen, wobei die Gesamtporosität einen Betrag von 85 Vol.-% nicht überschreitet.

3. Knochenaufbaumittel nach Anspruch 1 oder 2, **dadurch gekennzeichnet, dass** das Calciumphosphat im wesentlichen und insbesondere zu wenigstens 95 % aus Alpha-Tricalciumphosphat, Beta-Tricalciumphosphat, Octacalciumphosphat, alkali- und/oder erdalkalimodifiziertem Tricalciumphosphat, Calciumdiphosphat, Carbonatapatit vom B-Typ, Calcium-defizienten Hydroxylapatit oder deren Gemischen besteht.

4. Knochenaufbaumittel nach Anspruch 1 bis 3, **dadurch gekennzeichnet, dass** das Calciumphosphat vorzugsweise aus Beta-Tricalciumphosphat mit einer Phasenreinheit ≥ 99 Masse-%, bezogen auf die Fremdphase Hydroxylapatit besteht.

5. Knochenaufbaumittel nach Anspruch 1 bis 4, **dadurch gekennzeichnet, dass** es als Granulat ausgebildet ist und in verschiedenen Granulatfraktionen in einem Größenbereich zwischen 50 und 10000 µm vorliegt.

6. Knochenaufbaumittel gemäß Anspruch 5, **dadurch gekennzeichnet, dass** das Granulat eine im wesentlichen uneinheitliche geometrische Gestalt aufweist.

7. Knochenaufbaumittel gemäß Anspruch 5, **dadurch gekennzeichnet, dass** das Granulat eine im wesentlichen einheitliche geometrische Gestalt aufweist.

8. Knochenaufbaumittel gemäß Anspruch 7, **dadurch gekennzeichnet, dass** das Granulat im wesentlichen eine Kugelgestalt aufweist.

9. Knochenaufbaumittel gemäß Anspruch 5 bis 8, **dadurch gekennzeichnet, dass** die Maxima der diskreten Porengrößenverteilungen (II) oder (III) einen Betrag kleiner als die Hälfte der mittleren Granulatgröße einer Granulatfraktion aufweisen und vorzugsweise in einem Bereich zwischen 10 und 50% der mittleren Granulatgröße einer Granulatfraktion liegen.

10. Knochenaufbaumittel gemäß Anspruch 1, **dadurch gekennzeichnet, dass** es als Formkörper mit einem definierten geometrischen Design ausgebildet ist.

11. Knochenaufbaumittel gemäß Anspruch 10, **dadurch gekennzeichnet, dass** es zusätzlich zu einer statistischen Porosität eine gerichtete Porosität in Form von Röhrenporen aufweist.

12. Knochenaufbaumittel nach Anspruch 10 und 11, **dadurch gekennzeichnet, dass** die gerichtete Röhrenporosität durch maschinell eingebrachte ein-, zwei- oder dreidimensionale Bohrungen im Durchmesserbereich von 0,5 bis 2 mm ausgebildet ist und die Gesamtporosität aus statistischer und Röhrenporosität einen Wert von 85 Vol.-% nicht übersteigt.

13. Knochenaufbaumittel nach Anspruch 10 bis 12, **dadurch gekennzeichnet, dass** der kompakte Formkörper eine in Größe und Volumenanteil abgestufte Porengrößenverteilung vom Rand zum Kern hin aufweist, wobei vorzugsweise in der Randzone die Porengrößenverteilungen (I) und/oder (II), insbesondere mit einer Gesamtporosität bis zu 35 Vol.-%, in der Kernzone die Porengrößenverteilungen (I) und/oder (II) und/oder (III), insbesondere bis zu einer Gesamtporosität von 85 Vol.-%, vorliegen, wobei die Randzone einen Bereich von 10 % bis 40 % und die Kernzone von 60 % bis 90 % des Implantatgrößtmaßes senkrecht zur Zugspannungsrichtung oder parallel zur Biegebeanspruchung aufweist.

14. Knochenaufbaumittel nach einem der Ansprüche 1 bis 13, **dadurch gekennzeichnet, dass** es auf seiner Oberfläche und/oder in seiner inneren Porenstruktur antibakterielle, wundheilungsfördernde, knochenwachstumsfördernde und/oder gerinnungshemmende Substanzen in geeigneter, wirksamer Konzentration aufweist.

15. Knochenaufbaumittel nach einem der Ansprüche 10 bis 14, **dadurch gekennzeichnet, dass** es in standardisierten Abmessungen und Formen, vorzugsweise als Würfel, Quader, Zylinder oder Keil vorliegt.

16. Knochenaufbaumittel nach einem der Ansprüche 10 bis 14, **dadurch gekennzeichnet, dass** es eine indikationsbezogene Form, vorzugsweise als Trepanationsverschluss, Alveolaraugmentat oder Füllkörper für Cages zum Wirbelkörperersatz aufweist.

17. Verfahren zur Herstellung eines aus Calciumphosphat bestehenden Knochenaufbaumittels mit isotroper Sinterstruktur und statistisch verteilten Poren in mehreren diskreten Größenverteilungen über den Syntheseweg einer thermisch induzierten Festkörperreaktion aus an sich bekannten Ausgangsstoffen und Porosierungsmitteln, deren homogener Vermischung und Sinterung, **dadurch gekennzeichnet, dass** ein Calciumphosphat als Ausgangsstoff (C) aus seinen Ausgangsrohstoffen (A) und (B) synthetisiert wird, nachfolgend der synthetisierte Ausgangsstoff (C) auf eine Korngröße kleiner 63 µm mit einem d₅₀-Wert im Bereich von 5 bis 20 µm zerkleinert wird und mit einem Anteil seiner unumgesetzten Ausgangsstoffe (A) und (B) im stöchiometrischen Verhältnis versetzt wird, das Gemisch intensiv homogenisiert wird, nachfolgend das ausbrennbare oder sich verflüchtigende Porosierungsmittel Ammoniumhydrogencarbonat in Kornfraktionen mit d₅₀-Werten im Bereich von 0,5 bis 10 µm, 10 bis 100 µm und 100 bis 5000 µm zugesetzt werden, wobei die Kornfraktion des Porosierungsmittels mit einem d₅₀-Wert im Bereich von 0,5 bis 10 µm in einer Menge von 1 bis 20 Masse-%, die Kornfraktion des Porosierungsmittels mit einem d₅₀-Wert im Bereich von 10 bis 100 µm in einer Menge von 5 bis 40 Masse-% und die Kornfraktion Porosierungsmittels mit einem d₅₀-Wert im Bereich von 100 bis 5000 µm mit einer Menge im Bereich von 1 bis 40 Masse-%, jeweils bezogen auf die berechnete Menge calciumphosphat, zugesetzt wird; die Calciumphosphatbestandteile mit den Fraktionen der ausbrennbaren Porosierungsmittel ohne weitere Zerkleinerung homogenisiert und nachfolgend kompaktiert werden; die Porosierungsmittel durch Erhitzen entfernt werden; die porösen, ungebrannten Calciumphosphatkörper auf Reaktions-/Sintertemperatur für eine erforderliche Zeit erhitzt werden; die porösen Sinterkörper nachfolgend auf Raumtemperatur abgekühlt und in die gewünschte Form als Granulat oder Formteil gebracht werden.

18. Verfahren nach Anspruch 17, **dadurch gekennzeichnet, dass** als Porosierungsmittel Ammoniumhydrogencarbonat verwendet wird.

19. Verfahren nach Anspruch 17 oder 18, **dadurch gekennzeichnet, dass** dem synthetisierten Ausgangsstoff (C) das unumgesetzte Gemisch seiner unumgesetzten Ausgangsstoffe (A) und (B) im Molverhältnis 1:2 in einer Menge zwischen 1 und 50 Masse-%, bezogen auf die Menge an Ausgangsstoff (C), zugesetzt wird.

20. Verfahren nach einem der Ansprüche 17 bis 19, **dadurch gekennzeichnet, dass** es sich bei dem Ausgangsstoff (C) um Tricalciumphosphat, vorzugsweise ≥ 99 Masse-% phasenreines Beta-Tricalciumphosphat und bei den unumgesetzten Ausgangsstoffen (A) und (B) um Calciumcarbonat und Calciumhydrogenphosphat handelt.

21. Verfahren nach Anspruch 19 oder 20, **dadurch gekennzeichnet, dass** die Kompaktierung der Mischung des synthetisierten Ausgangsstoffes (C), des Anteils des Gemisches seiner unumgesetzten Ausgangsstoffe (A) und (B) im Molverhältnis 1:2 sowie der Porosierungsmittel isostatisch bei einem Pressdruck von 100 bis 250 MPa erfolgt.

22. Verfahren nach einem der Ansprüche 19 bis 21, **dadurch gekennzeichnet, dass** die kompaktierte Mischung des synthetisierten Ausgangsstoffes (C), der Mischung seiner unumgesetzten Ausgangsstoffe (A) und (B) im Molverhältnis 1:2 sowie des ausbrennbaren Porosierungsmittels mit einer Aufheizgeschwindigkeit im Bereich von 0,5 bis 5 K/min in den Bereich von 1373 bis 1573 K erhitzt, bei dieser Temperatur vorzugsweise 24 bis 72 Stunden gehalten und anschließend mit einer Abkühlgeschwindigkeit von 0,5 bis 5 K/min wieder auf Raumtemperatur abgekühlt wird.

23. Verfahren nach Anspruch 22, **dadurch gekennzeichnet, dass** bei der kontrollierten Temperaturbehandlung eine zusätzliche Temperatur-Haltestufe im Bereich von 1123 bis 1223 K verwendet wird.

## Claims

1. Bone formation agent of porous calcium phosphate having an isotropic sintered structure and, between the sintered particles of the calcium phosphate, statistically distributed pores in a plurality of discrete size ranges, **characterised in that** it has a porosity composed of three discrete pore size distributions (I), (II) and (III), and the maxima of the three discrete pore size distributions are at pore diameters in the intervals from 0.5 to 10 µm (I), 10 to 100 µm (II) and 100 to 5000 µm (III), the porosity has an irregular geometric shape, the sintered particles of the calcium phosphate have a particle size smaller than 63 µm with a d₅₀ value in the range from 5 to 20 µm, and the interconnecting pore share in the porosity is limited to pore sizes less than 10 µm; obtainable by synthesising a calcium phosphate as starting material (C) from its starting raw materials (A) and (B), the synthesised starting material (C) is subsequently comminuted to a particle size smaller than 63 µm with a d₅₀ value in the range from 5 to 20 µm and a proportion of its unreacted starting raw materials (A) and (B) in a stoichiometric ratio is added, the mixture is intensively homogenised and subsequently the porosity-causing agent ammonium hydrogen carbonate, that can be burnt off or that is volatilized, is added in particle fractions having d₅₀ values in the range from 0.5 to 10 µm, 10 to 100 µm and 100 to 5000 µm, wherein the particle fraction of the porosity-causing agent having a d₅₀ value in the range from 0.5 to 10 µm is added in an amount of from 1 to 20% by weight, the particle fraction of the porosity-causing agent having a d₅₀ value in the range from 10 to 100 µm is added in an amount of from 5 to 40% by weight and the particle fraction of the porosity-causing agent having a d₅₀ value in the range from 100 to 5000 µm is added in an amount of from 1 to 40% by weight, each based on the calculated amount of calcium phosphate; the calcium phosphate constituents are homogenised together with the fractions of the porosity-causing agents that can be burnt off, without further comminution, and are subsequently compacted; the porosity-causing agents are removed by heating; the porous non-fired calcium phosphate bodies are heated to reaction/sintering temperature for a requisite time; the porous sintered bodies are subsequently cooled down to room temperature and brought into the desired form of a granulate or shaped part.

2. Bone formation agent according to claim 1, **characterised in that** the volume shares of the discrete pore size distributions (I) to (III) are in the range from 20 to 40% by volume for pore size distribution (I), in the range from 5 to 40% by volume for pore size distribution (II) and in the range from 1 to 40% by volume for pore size distribution (III), the overall porosity not exceeding a value of 85% by volume.

3. Bone formation agent according to claim 1 or 2, **characterised in that** the calcium phosphate consists to a substantial extent, and especially to an extent of at least 95%, of alpha-tricalcium phosphate, beta-tricalcium phosphate, octacalcium phosphate, alkali metal-modified and/or alkaline earth metal-modified tricalcium phosphate, calcium diphosphate, carbonate apatite of type B, calcium-deficient hydroxyapatite or mixtures thereof.

4. Bone formation agent according to claim 1 to 3, **characterised in that** the calcium phosphate consists preferably of beta-tricalcium phosphate having a phase purity of ≥ 99% by weight, relative to the foreign hydroxyapatite phase.

5. Bone formation agent according to claim 1 to 4, **characterised in that** it is in the form of a granulate and is present in various granulate fractions in a size range between 50 and 10000 µm.

6. Bone formation agent according to claim 5, **characterised in that** the granulate has a substantially non-uniform geometric shape.

7. Bone formation agent according to claim 5, **characterised in that** the granulate has a substantially uniform geometric shape.

8. Bone formation agent according to claim 7, **characterised in that** the granulate has a substantially spherical shape.

9. Bone formation agent according to claim 5 to 8, **characterised in that** the maxima of the discrete pore size distributions (II) or (III) are a value less than half the average granulate size of a granulate fraction and are preferably in a range between 10 and 50% of the average granulate size of a granulate fraction.

10. Bone formation agent according to claim 1, **characterised in that** it is in the form of a shaped body having a defined geometric design.

11. Bone formation agent according to claim 10, **characterised in that** in addition to a statistical porosity it has a defined porosity in the form of tubular pores.

12. Bone formation agent according to claim 10 and 11, **characterised in that** the defined tubular porosity is formed by one-, two- or three-dimensional bores, introduced by machining, in the diameter range from 0.5 to 2 mm, and the overall porosity consisting of statistical and tubular porosity does not exceed a value of 85% by volume.

13. Bone formation agent according to claim 10 to 12, **characterised in that** the compact shaped body has a pore size distribution graduated in size and volume share from the periphery to the core, with preferably in the peripheral zone pore size distributions (I) and/or (II) being present, especially with an overall porosity of up to 35% by volume, and in the core zone pore size distributions (I) and/or (II) and/or (III) being present, especially up to an overall porosity of 85% by volume, with the peripheral zone having a range from 10% to 40% and the core zone from 60% to 90% of the largest dimension of the implant perpendicular to the tensile stress direction or parallel to the bending stress.

14. Bone formation agent according to any one of claims 1 to 13, **characterised in that** it has, on its surface and/or in its internal pore structure, antibacterial, wound healing-promoting, bone growth-promoting and/or anticoagulant substances in suitable effective concentrations.

15. Bone formation agent according to any one of claims 10 to 14, **characterised in that** it is present in standardised dimensions and shapes, preferably in the form of a cube, cuboid, cylinder or wedge.

16. Bone formation agent according to any one of claims 10 to 14, **characterised in that** it has an indication-related shape, preferably in the form of a trepanation closure, alveolar augmentation or filler for cages for vertebrae replacement.

17. Method of producing a bone formation agent consisting of calcium phosphate having an isotropic sintered structure and statistically distributed pores in a plurality of discrete size distributions by way of the synthesis route of a thermally induced solid-state reaction beginning with starting materials and porosity-causing agents that are known per se, their homogeneous mixing and sintering, **characterised in that** a calcium phosphate as starting material (C) is synthesised from its starting raw materials (A) and (B), the synthesised starting material (C) is subsequently comminuted to a particle size smaller than 63 µm with a d₅₀ value in the range from 5 to 20 µm and a proportion of its unreacted starting raw materials (A) and (B) in a stoichiometric ratio is added, the mixture is intensively homogenised and subsequently the porosity-causing agent ammonium hydrogen carbonate that can be burnt off or that is volatilised is added in particle fractions having d₅₀ values in the range from 0.5 to 10 µm, 10 to 100 µm and 100 to 5000 µm, wherein the particle fraction of the porosity-causing agent having a d₅₀ value in the range from 0.5 to 10 µm is added in an amount of from 1 to 20% by weight, the particle fraction of the porosity-causing agent having a d₅₀ value in the range from 10 to 100 µm is added in an amount of from 5 to 40% by weight and the particle fraction of the porosity-causing agent having a d₅₀ value in the range from 100 to 5000 µm is added in an amount of from 1 to 40% by weight, each based on the calculated amount of calcium phosphate; the calcium phosphate constituents are homogenised together with the fractions of the porosity-causing agents that can be burnt off, without further comminution, and are subsequently compacted; the porosity-causing agents are removed by heating; the porous non-fired calcium phosphate bodies are heated to reaction/sintering temperature for a requisite time; the porous sintered bodies are subsequently cooled down to room temperature and brought into the desired form of a granulate or shaped part.

18. Method according to claim 17, **characterised in that** ammonium hydrogen carbonate is used as porosity-causing agent.

19. Method according to claim 17 or 18, **characterised in that** to the synthesised starting material (C) there is added a proportion of the mixture of its unreacted starting materials (A) and (B) in a molar ratio of 1:2 in an amount between 1 and 50% by weight, based on the amount of starting material (C).

20. Method according to any one of claims 17 to 19, **characterised in that** the starting material (C) is tricalcium phosphate, preferably beta-tricalcium phosphate having a phase purity of ≥99% by weight, and the unreacted starting raw materials (A) and (B) are calcium carbonate and calcium hydrogen phosphate.

21. Method according to claim 19 or 20, **characterised in that** the compacting of the mixture of the synthesised starting material (C) with the proportion of the mixture of its unreacted starting raw materials (A) and (B) in a molar ratio of 1:2 and the porosity-causing agents is carried out isostatically under a compression pressure of from 100 to 250 MPa.

22. Method according to any one of claims 19 to 21, **characterised in that** the compacted mixture of the synthesised starting material (C) with the mixture of its unreacted starting raw materials (A) and (B) in a molar ratio of 1:2 and the porosity-causing agent that can be burnt off is heated at a heating rate in the range from 0.5 to 5 K/min to the range from 1373 to 1573 K, is held at that temperature for preferably from 24 to 72 hours and is then cooled back down to room temperature at a cooling rate of from 0.5 to 5 K/min.

23. Method according to claim 22, **characterised in that**, in the controlled temperature treatment, an additional temperature-holding step in the range from 1123 to 1223 K is used.

## Revendications

1. Agent d'ostéogénèse constitué de phosphate de calcium poreux comprenant une structure frittée isotrope et des pores statistiquement distribuées entre les particules frittées en plusieurs distributions de tailles discrètes, **caractérisé en ce qu'**il présente une porosité composée de trois distributions de tailles de pores discrètes (I), (II) et (III) et que les valeurs maximales des trois distributions de tailles de pores discrètes se situent pour des diamètres de pores dans les intervalles de 0,5 à 10 µm (I), de 10 à 100 µm (II) et de 100 à 5000 µm (III), que la porosité possède une forme géométrique irrégulière, que les particules frittées de phosphate de calcium présentent une taille particulaire inférieure à 63 µm avec une valeur d₅₀ dans le domaine de 5 à 20 µm et la proportion de pores interconnectées de la porosité est limitée à des tailles de pores inférieures à 10 µm ; que l'on peut obtenir en synthétisant un phosphate de calcium en tant que produit de départ (C) à partir de ses matières premières (A) et (B), ensuite, le produit de départ synthétisé (C) est divisé pour avoir une taille granulaire inférieure à 63 µm avec une valeur d₅₀ dans le domaine de 5 à 20 µm et est mis à réagir dans un rapport stoechiométrique avec une partie de ses matières premières (A) et (B) non converties, le mélange est intensivement homogénéisé, ensuite, l'agent porogène, l'hydrogénocarbonate d'ammonium, pouvant être grillé ou se volatilisant, est ajouté sous la forme de fractions granulaires avec des valeurs d₅₀ dans le domaine de 0,5 à 10 µm, de 10 à 100 µm et de 100 à 5000 µm, les fractions granulaires de l'agent porogène avec une valeur d₅₀ dans le domaine de 0,5 à 10 µm étant ajoutées en une quantité de 1 à 20 % en masse, les fractions granulaires de l'agent porogène avec une valeur d₅₀ dans le domaine de 10 à 100 µm étant ajoutées en une quantité de 5 à 40 % en masse et les fractions granulaires de l'agent porogène avec une valeur d₅₀ dans le domaine de 100 à 5000 µm étant ajoutées en une quantité de 1 à 40 % en masse, chacune par rapport à la quantité calculée de phosphate de calcium ; les constituants du phosphate de calcium sont homogénéisés avec les fractions de l'agent porogène pouvant être grillé sans division complémentaire et sont ensuite compactées ; les agents porogènes sont enlevés par un chauffage ; les corps de phosphate de calcium poreux non grillés sont réchauffés à une température de réaction-frittage pendant une durée nécessaire ; les corps frittés poreux sont ensuite refroidis à la température ambiante et sont mis sous la forme souhaitée en tant que granulés ou pièce moulée.

2. Agent d'ostéogénèse selon la revendication 1, **caractérisé en ce que** les proportions en volume des distributions de tailles des pores discrètes (I) à (III) se situent dans le domaine de 20 à 40 % en volume pour la distribution des tailles des pores (I), dans le domaine de 5 à 40 % en volume pour la distributions des tailles des pores (II) et dans le domine de 1 à 40 % en volume pour la distribution des tailles des pores (III), la porosité totale ne dépassant pas une valeur de 85 % en volume.

3. Agent d'ostéogénèse selon la revendication 1 ou 2, **caractérisé en ce que** le phosphate de calcium est constitué essentiellement et notamment d'au moins 95 % de phosphate alpha tricalcique, de phosphate bêta tricalcique, de phosphate octacalcique, de phosphate tricalcique modifié par un alcalin et/ou un alcalino-terreux, de diphosphate de calcium, de carbonate-apatite de type B, d'hydroxy-apatite déficiente en calcium ou de leurs mélanges.

4. Agent d'ostéogénèse selon la revendication 1 à 3, **caractérisé en ce que** le phosphate de calcium est constitué de préférence de phosphate bêta tricalcique avec une homogénéité de phases ≥ 99 % en masse par rapport à la phase étrangère d'hydroxy-apatite.

5. Agent d'ostéogénèse selon la revendication 1 à 4, **caractérisé en ce qu'**il est conçu sous la forme de granulé et se présente en différentes fractions granulaires dans un domaine de tailles entre 50 et 10000 µm.

6. Agent d'ostéogénèse selon la revendication 5, **caractérisé en ce que** le granulé présente une forme géométrique essentiellement hétérogène.

7. Agent d'ostéogénèse selon la revendication 5, **caractérisé en ce que** le granulé présente une forme géométrique essentiellement homogène.

8. Agent d'ostéogénèse selon la revendication 7, **caractérisé en ce que** le granulé présente essentiellement une forme sphérique.

9. Agent d'ostéogénèse selon la revendication 5 à 8, **caractérisé en ce que** les valeurs maximales des distributions des tailles des pores discrètes (II) ou (III) représentent une valeur inférieure à la moitié des tailles des granulés moyennes d'une fraction de granulés et se situent de préférence dans un domaine entre 10 et 50 % des tailles moyennes des granulés d'une fraction de granulés.

10. Agent d'ostéogénèse selon la revendication 1, **caractérisé en ce qu'**il est sous la forme d'un corps moulé avec un stylisme géométrique défini.

11. Agent d'ostéogénèse selon la revendication 10, **caractérisé en ce qu'**il se présente avec une porosité orientée sous la forme de pores tubulaires complémentairement à une porosité statistique.

12. Agent d'ostéogénèse selon la revendication 10 et 11, **caractérisé en ce que** la porosité tubulaire orientée est formée par des perçages uni-, bi- ou tridimensionnels dans un domaine de diamètres de 0,5 à 2 mm rapportés mécaniquement et que la porosité totale constituée par la porosité statistique et par la porosité tubulaire n'excède pas une valeur de 85 % en volume.

13. Agent d'ostéogénèse selon la revendication 10 à 12, **caractérisé en ce que** le corps moulé compact présente une distribution de tailles de pores échelonnée en taille et en proportion volumique entre le bord et le coeur, les distributions de tailles de pores (I) et/ou (II) étant de préférence présentes dans la zone du bord, notamment avec une porosité totale jusqu'à 35 % en volume, les distributions de tailles de pores (I) et/ou (II) et/ou (III) étant présentes dans la zone de coeur, notamment avec une porosité totale jusqu'à 85 % en volume, la zone du bord présentant un domaine de 10 % à 40 % de la plus grande partie des implantations dans la zone de bord et de 60 % à 90 % de la plus grande partie des implantations dans la zone de coeur perpendiculaire à la direction de la contrainte en traction ou parallèle à la sollicitation en flexion.

14. Agent d'ostéogénèse selon l'une des revendications 1 à 13, **caractérisé en ce qu'**il présente sur sa surface et/ou dans sa structure poreuse interne des substances antibactériennes, favorisant la cicatrisation, favorisant la croissance osseuse et/ou anticoagulantes à une concentration appropriée, efficace.

15. Agent d'ostéogénèse selon l'une des revendications 10 à 14, **caractérisé en ce qu'**il se présente dans des dimensions et des formes standardisées, de préférence sous forme d'un cube, d'un parallélépipède rectangle, d'un cylindre ou d'un cône.

16. Agent d'ostéogénèse selon l'une des revendications 10 à 14, **caractérisé en ce qu'**il présente une forme relative à une indication, de préférence sous la forme d'une fermeture de trépanation, d'une augmentation alvéolaire ou d'un produit de remplissage de cages pour un substitut de corps vertébral.

17. Procédé de fabrication d'un agent d'ostéogénèse constitué de phosphate de calcium avec une structure frittée isotrope et des pores statistiquement distribuées en plusieurs distributions de tailles discrètes par l'intermédiaire d'une voie de synthèse par une réaction des corps à l'état solide induite thermiquement à partir de produits de départ bien connus et d'agents porogènes, dont le mélange homogène et le frittage sont **caractérisés en ce qu'**un phosphate de calcium est synthétisé en tant que produit de départ (C) à partir des matières premières (A) et (B), ensuite, le produit de départ synthétisé (C) est divisé pour avoir une taille granulaire inférieure à 63 µm avec une valeur d₅₀ dans le domaine de 5 à 20 µm et mis à réagir dans un rapport stoechiométrique avec une partie de ses matières premières (A) et (B) non converties, le mélange est homogénéisé intensivement, ensuite, l'agent porogène, l'hydrogénocarbonate d'ammonium, pouvant être grillé ou se volatilisant, est ajouté sous la forme de fractions granulaires avec des valeurs d₅₀ dans le domaine de 0,5 à 10 µm, de 10 à 100 µm et de 100 à 5000 µm, les fractions granulaires de l'agent porogène avec une valeur d₅₀ dans le domaine de 0,5 à 10 µm étant ajoutées en une quantité de 1 à 20 % en masse, les fractions granulaires de l'agent porogène avec une valeur d₅₀ dans le domaine de 10 à 100 µm étant ajoutées en une quantité de 5 à 40 % en masse et les fractions granulaires de l'agent porogène avec une valeur d₅₀ dans le domaine de 100 à 5000 µm étant ajoutées en une quantité de 1 à 40 % en masse, chacune par rapport à la quantité calculée de phosphate de calcium ; les constituants du phosphate de calcium sont homogénéisés avec les fractions de l'agent porogène pouvant être grillé sans division complémentaire et sont ensuite compactés ; les agents porogènes sont enlevés par un chauffage ; les corps de phosphate de calcium poreux non grillés sont réchauffés à une température de réaction-frittage pendant la durée nécessaire ; les corps frittés poreux sont ensuite refroidis à la température ambiante et sont mis sous la forme souhaitée en tant que granulés ou corps moulées.

18. Procédé selon la revendication 17, **caractérisé en ce que** de l'hydrogénocarbonate d'ammonium est employé en tant qu'agent porogène.

19. Procédé selon la revendication 17 ou 18, **caractérisé en ce qu'**au produit de départ synthétisé (C), le mélange non converti de ses matières premières (A) et (B) non converties est ajouté dans la proportion molaire 1 : 2 en une quantité entre 1 et 50 % en masse, par rapport à la quantité de produit de départ (C).

20. Procédé selon l'une des revendications 17 à 19, **caractérisé en ce qu'**il s'agit de phosphate tricalcique, de préférence, de phosphate bêta tricalcique de phase pure à ≥ 99 % en masse pour le produit de départ (C), et de carbonate de calcium et d'hydrogénophosphate de calcium pour les matières premières (A) et (B) non converties.

21. Procédé selon la revendication 19 ou 20, **caractérisé en ce que** le compactage du mélange du produit de départ (C) synthétisé, de la proportion dans le mélange de ses matières premières (A) et (B) non converties dans le rapport molaire 1 : 2 ainsi que des agents porogènes s'effectue de manière isostatique avec une pression de compression de 100 à 250 MPa.

22. Procédé selon l'une des revendications 19 à 21, **caractérisé en ce que** le mélange compacté du produit de départ (C) synthétisé, du mélange de ses matières premières (A) et (B) non converties dans un rapport molaire 1 : 2, ainsi que de l'agent porogène pouvant être grillé est chauffé avec une vitesse d'élévation de chauffage dans le domaine de 0,5 à 5 K/min dans une gamme de température de 1373 à 1573 K, est maintenu à cette température de préférence de 24 à 72 heures et est ensuite de nouveau refroidi avec une vitesse de refroidissement de 0,5 à 5 K/min jusqu'à la température ambiante.

23. Procédé selon la revendication 22, **caractérisé en ce qu'**une étape de maintien en température complémentaire dans le domaine entre 1123 et 1223 K est employée lors du traitement thermique contrôlé.
